(19) Europäisches Patentamt
European Patent Office
Office européen des brevets

(11) EP 4 659 830 A2

(12) EUROPEAN PATENT APPLICATION

(43) Date of publication:
**10.12.2025 Bulletin 2025/50**

(21) Application number: **25211270.1**

(22) Date of filing: **24.06.2021**

(51) International Patent Classification (IPC):
***B01D 11/02*** *(2006.01)*

(52) Cooperative Patent Classification (CPC):
(C-Sets available)
**B01D 11/0492; B01D 9/0013; B01J 27/232; B01J 31/0202; B01J 31/0204; B01J 31/0212; B01J 31/0251; B01J 31/04; C07C 67/03; C07C 67/54; C07C 67/58; C08J 11/22; C08J 11/24;**
B01J 2231/49; C08J 2367/02; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2020 KR 20200078652**
**19.01.2021 KR 20210007240**
**26.02.2021 KR 20210026879**

(62) Document number(s) of the earlier application(s) in accordance with Art. 76 EPC:
**21827959.4 / 4 155 342**

(71) Applicant: **Korea Research Institute of Chemical Technology**
**Daejeon 34114 (KR)**

(72) Inventors:
- **CHO, Joungmo**
**34114 Daejeon (KR)**
- **HONG, Do Young**
**34114 Daejeon (KR)**
- **LE, Thi Hong Ngan**
**34114 Daejeon (KR)**

(74) Representative: **Isarpatent**
**Patent- und Rechtsanwälte Partg mbB**
**Friedrichstraße 31**
**80801 München (DE)**

Remarks:
This application was filed on 27-10-2025 as a divisional application to the application mentioned under INID code 62.

# (54) EFFICIENT DEPOLYMERIZATION METHOD OF POLYMER COMPRISING ESTER FUNCTIONAL GROUP, AND PURIFICATION METHOD THEREOF

(57) The present disclosure relates to a depolymerization method of a polymer comprising an ester functional group with or without pigments or dyes, a composition for the depolymerization, and a purification method for selectively separating foreign substances from a monomer of the polymer, and is characterized in that a compound represented by formula 1 is added as an additive. The depolymerization method according to the present disclosure enables depolymerization at low temperatures and may increase the yield of a target product. **In** addition, the purification method according to the present disclosure may remove foreign substances including pigments or dyes from a monomer of the polymer to prepare high-purity monomers.

EP 4 659 830 A2

(52) Cooperative Patent Classification (CPC): (Cont.)
Y02W 30/62

C-Sets
**C07C 67/03, C07C 69/82;**
**C07C 67/54, C07C 69/82;**
**C07C 67/58, C07C 69/82**

## Description

### Technical Field

The present disclosure relates to a method for low-temperature glycolysis of an ester functional group-bearing polymer in the presence of an additive thereto, a method for removal of foreign matter capable of being incorporated into monomers from the depolymerized product of a colored polymer and a method for purifying monomers in high purity, using same, and a method for eco-friendly depolymerization of the colored polymer.

More specifically, the present disclosure relates to a method for eco-friendly depolymerization of an ester functional group-bearing colored polymer through glycolysis wherein a certain additive is used, whereby a high level of depolymerization yield can be obtained even at a low temperature, a method for selectively separating colored foreign materials through liquid-liquid phase equilibration by adjusting thermodynamic conditions for the obtained reaction mixture, a method for purifying highly valuable monomers in high purity through liquid-liquid extraction, a method for depolymerization of an ester functional group-bearing colored polymer, in which a monomeric product prepared in the presence of an inexpensive transesterification catalyst bearing no harmful metals can greatly decrease in the content of organic or inorganic impurities that are harmful or degrade product quality, and a method for purifying a monomeric product in high purity.

### Background Art

Most discarded plastics which are difficult to decompose naturally are randomly discharged to designated landfills or natural habitats, making environmental pollution problems more serious day by day. Even biodegradable or biodegradable plastics can persist for decades, depending on local environmental factors such as level of UV exposure, temperature, presence of degrading microorganisms, etc.

To solve these problems, various studies have been being in progress, which target the development of novel plastic materials decomposable within a short period of time in nature, covering a broad spectrum ranging from the chemical degradation of existing petroleum-based plastic to the physical recycling and reprocessing of plastics, with the aim of minimizing plastic accumulation and reducing the influence of plastics on the environment.

Polymers bearing ester functional groups can be monomerized through depolymerization, and various chemical reaction pathways have been developed. The monomers generated through decomposition can theoretically have properties equivalent to those of the raw materials used in the initial polymer synthesis. Depolymerization pathways industrially applied to recycle polyester include hydrolysis, glycolysis, methanolysis, ammonolysis, etc., and use is widely made of various chemical depolymerization methods including complex processes that take a combination of only advantages by process from the depolymerization pathways.

For more details of the above-mentioned methods for depolymerization of ester functional group-bearing polymers, first, it is known that hydrolysis can proceed through various pathways in the presence of an acidic, base, or metal salt catalyst. An acidic catalyst reaction requires a very high concentration of a sulfuric acid solution for acquiring a high reaction yield, which leads to disadvantages such as economic problems in process design, operation, and post-processing. Employment of a base or a metal salt catalyst is inefficient because of the problems that the reaction proceeding slowly, the product is low in purity, and it is difficult to recover the catalyst.

The methanolysis process is one of the processes widely applied to actual commercial processes, not only in global chemical companies but also in small and medium-sized plastic industries. However, the use of methanol as a reaction solvent requires severe reaction conditions of high temperature and high pressure, incurring the cost of accompanying equipment design, and additional unit processes for reactant recovery and product purification are also essential, which results in requirement of a high investment cost. Effective methanolysis may be performed in the presence of a transesterification catalyst. Catalysts generally used in the depolymerization reaction of polymers bearing ester functional groups contain metals as cations, as exemplified by zinc acetate, magnesium acetate, cobalt acetate, and lead dioxide. However, the metal constituent of such a catalyst may remain in the depolymerization product obtained. Thus, the catalysts are subject to restrictions that must be reviewed together with the efficiency and economic feasibility of the reaction and purification process with respect to the type and amount of the catalysts in order to minimize the effects of catalysts on human health or product quality depending on the use and purpose of the material being repolymerized.

Hydrolysis and methanolysis processes are used in various fields. However, both the reactions exhibit limited quality and yields of the products obtained by decomposition of polyester resins and proceed for a long time until completion of the decomposition. After completion of each of the reactions, only one of terephthalic acid (TPA) or dimethylterephthalate (DMT) is produced as the main product while other types of terephthalate compounds are treated as by-product impurities. Thus, in order to re-synthesize the obtained product into a high-quality polymer material, a refining process with excessive energy consumption may be required.

Glycolysis is a depolymerization reaction in which glycol is added as a reactant. The most common example of

glycolysis is a process for preparing bis(2-hydroxyethyl) terephthalate (BHET) by adding an excess of ethylene glycol (EG), one of the monomer raw materials. Because ethylene glycol, which is a part of PET raw material, is used as a reactant, the process enjoys the advantages of being high in thermodynamic miscibility with the reaction product and being able to be derived by a modification to an existing polymerization process at only a small investment in facility and to apply raw materials directly to production without additional chemical treatment. Glycolysis is generally carried out under the reflux condition of glycol. In glycolysis, the polymer decomposition step rate is slow and the product shows a broad range of distributions even in the reaction equilibrium state, so that the product may have limits to not only the reaction yield but also the production yield. In order to separate the monomer, which is the final product, from the reactants in high yield and high purity, a high-cost purification process may be essential. General catalysts used in the process include zinc acetate and lithium acetate as disclosed in Korean Patent No. 2012-01228480 A and Korean Patent No. 1142328. These metal salt catalysts are not completely removed during the purification process and may remain in the product, and even a small amount of metal that is harmful to the human body might be contained in the regenerated monomers. If so, the monomers are not suitable as raw materials for products prepared therefrom, especially, goods in the food and medical fields and household goods that require harmlessness. In addition, although glycolysis proceeds at a high temperature, the recovery and purification of the product is generally carried out according to a low-temperature recrystallization method. Thus, there is a problem in that energy consumption is high and the heat source supply method of the production process is poor in terms of cost and efficiency.

Application of a catalyst system that can increase depolymerization reaction rates at low temperatures and is of low toxicity is expected to not only drive the glycolysis reaction in an efficient and economical manner, but also allow mass-discharged ester functional group-bearing polymers to be effectively prepared into eco-friendly raw materials that do not impart limitations to the quality and uses of the products prepared therefrom.

Monomers produced through chemical decomposition such as depolymerization can theoretically be produced with properties equivalent to those of the raw materials used in the initial polymer synthesis or with a purity that can be used for polymer polymerization. However, waste polymer materials discarded after use contain various types of foreign substances, which can provide the biggest cause of deteriorating the quality of final products.

In particular, since coloring compound, such as dyes and pigments, that penetrate directly into polymer substrates are incorporated into products through a series of physical and chemical pre- and post-treatments and coloring processes as well as material design to prevent discoloration, the coloring compounds are difficult to remove by a simple washing or cleaning process. It is also not easy to chemically separate or isolate the coloring compounds even from the products of depolymerization, that is, low-molecular weight compounds containing a terephthalic acid structure.

Pigments, which are dispersed in the form of powder in a solvent, are characterized by being insoluble in water or oil while dyes can be uniformly soluble in most solvents and can have very small particle sizes or molecular forms. Pigments are mostly introduced into polymer resins by painting or printing methods, and dyes are mostly applied to dyeing textiles or leather through an exhaustion and fixation process. Dyes are further classified into natural dyes obtained from plants, animals, or minerals and artificial dyes synthesized artificially. Examples of artificial dyes include direct dyes, mordant dyes, reducing dyes, chromogenic dyes, dispersible dyes, reactive dyes, and the like. Dyes mainly applied to ester functional group-bearing polymeric materials may be representatively exemplified by azo- and anthraquinone-based dyes according to the classification by chemical structure.

When obtaining high-value monomers by purifying the depolymerization product of an ester functional group-bearing polymers, related arts for producing pigment-removed, high-purity monomers includes an adsorption method using an adsorbent such as activated carbon, a physical separation method such as filtration or distillation, and chemical methods such as oxidation, reduction, hydrolysis, and electrolysis. However, when remaining in the product, even a trace amount of a dye compound expresses a color, and the effect is often very fragmentary due to the coloring property of forming a complex with polymer monomers. Furthermore, since the cost of materials and energy applied to the separation process may be excessive, it is very difficult to secure economic feasibility in most cases.

With respect to related art, reference may be made to Japanese Patent No. 4537288 that discloses a method for preparing dimethyl terephthalate and alkylene glycol from dye-colored polyester fibers, including a dye extraction process, a solid-liquid separation process, a depolymerization process, a transesterification process, and an active ingredient recovery process. In this patent document, xylene and alkylene glycol are used as extraction solvents to extract a dye from a dyed polyester fiber, and the dye is extracted and removed at a temperature from higher than the glass transition point of the polyester to 220°C. The dye is extracted before depolymerization, and then the dye-removed polyester fiber is added with alkylene glycol and some depolymerization reaction solution and subjected to depolymerization in the presence of a depolymerization catalyst to obtain a depolymerized solution containing bis-ω-hydroxyalkyl terephthalate (BHAT). However, extracting a dye before depolymerization is uneconomical and undesirable because the extraction efficiency is low.

In addition, Korean Patent No. 10-1142329 discloses a technique including the steps of contacting an oligomer solution obtained by the depolymerization of a polyester with an ion exchange resin to remove a catalyst from the oligomer solution and passing the catalyst-removed oligomer solution through a decoloring agent, such as active carbon, activated

clay, diatomite, etc., to remove colorants from the oligomer solution through adsorption into the decoloring agent. However, the method of the above patent is not economically beneficial because the amount of the decoloring agent may be excessive and regeneration of the adsorbent is limited.

In order to solve the problems encountered in the related art, there is a need for development of materials or processes that can effectively remove dyes or pigments from terephthalate derivatives generated during the depolymerization of ester functional group-bearing colored polymers or the product purification process, without excessive energy consumption.

## Disclosure

### Technical Problem

According to the present disclosure, ester functional group-bearing polymers are decomposed in such a controlled manner that the resulting monomers are equivalent to the initial raw materials before polymerization or raw materials prepared in the petrochemical process or have a very low impurity content. In addition, the present disclosure provides a glycolysis-based method for manufacturing a high-quality, regenerated monomer, wherein waste polymers discarded after consumption are used as materials for decomposition and the monomer prepared therefrom can be re-fed to the same process in the same process without any additional pre-treatment process, whereby the method can make a contribution to plastic recycling economy.

In addition, the present disclosure is to provide a depolymerization method that allows for decomposition at much lower temperatures than conventional high-temperature reaction conditions, thereby significantly reducing energy consumption.

Furthermore, the present disclosure is to provide a method for manufacturing monomers, which is designed to employ eco-friendly reaction substances, reaction routes, and system configurations, compared to conventional processes and enables easy purification of products depolymerized from ester-functional group-bearing polymers, whereby the monomers can be obtained economically and efficiently, compared to other reaction techniques.

Also provided herein are an extractant that is added to a monomer of an ester functional group-bearing polymer to selectively extract the foreign impurity colorant present in the monomer and a method for purifying monomers of an ester functional group-bearing polymer by using same.

Moreover, the present disclosure is to provide a method for purifying monomers from a reaction mixture or monomer mixture generated during a depolymeization process of a polymer, wherein purification is performed either in-situ in the same process used in the depolymerization or through an additional post-treatment process so as for the monomers to be prepared from to the same quality level of those before the polymerization.

Furthermore, the present disclosure is to provide a method for producing a high-purity monomer or derived compound of a polymer, wherein an extraction process is repeated while a small amount of an additive is added, thereby controlling the purity of the product.

### Technical Solution

An aspect of the present disclosure provides a method for depolymerization of an ester functional group-bearing polymer, the method including contacting with the ester functional group-bearing polymer a mixture including: (1) at least one polyhydric alcohol; (2) a transesterification catalyst; and (3) a compound represented by the following Chemical Formula 1:

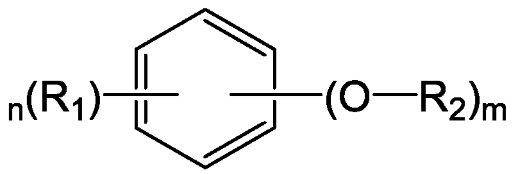

[Chemical Formula 1]

wherein,

$R_1$ is any one selected from the group consisting of a hydrogen atom, a hydroxy, an aldehyde, a carboxyl, an alkyl of $C_1$-$C_6$, a cycloalkyl of $C_4$-$C_6$, and an aryl of $C_6$-$C_{12}$;
n is an integer of 0 to 5 wherein when n is 2 or more, the corresponding $R_1$'s are same or different;
$R_2$ is an alkyl of $C_1$-$C_{10}$; and
m is an integer of 1 to 6 wherein when m is 2 or more, the corresponding -O-$R_2$'s are same or different.

In an embodiment of the present disclosure, the transesterification catalyst may include at least one metal salt selected from among salts of metals in groups 1A, 2A, and 2B.

In an embodiment of the present disclosure, the metal salt may include a counter ion composed of an organic anion selected from among carbonate, bicarbonate, alkoxide, and acetate.

In an embodiment of the present disclosure, the polyhydric alcohol may be at least one selected from among ethylene glycol, diethylene glycol, triethylene glycol, propylene glycol, dipropylene glycol, 1,3-butanediol, 1,4-butanediol, 3-methyl-1,5-pentanediol, 1,6-hexanediol, 1,7-octanediol, 1,9-nonanediol, neopentyl glycol, 1,4-cyclohexanediol, isosorbide, and 1,4-cyclohexane dimethanol.

In an embodiment of the present disclosure, the ester functional group-bearing polymer may be at least one selected from the group consisting of polyethylene terephthalate (PET), polypropylene terephthalate (PPT), polyglycolide or polyglycolic acid (PGA), polylactic acid (PLA), polycaprolactone (PCL), polyhydroxyalkanoate (PFM), polyhydroxybutyrate (PHB), polyethylene adipate (PEA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), and vectran.

In an embodiment of the present disclosure, the compound represented by Chemical Formula 1 may be at least one selected from the group consisting of methoxybenzene, 1,2-dimethoxybenzene, 1,3-dimethoxybenzene, 1,4-dimethoxybenzene, 1,2,3-trimethoxybenzene, 1,2,4-trimethoxybenzene, 1,3,5-trimethoxybenzene, 1,2,3,4-tetramethoxybenzene, 1,2,3,5-tetramethoxybenzene, 1,2,4,5-tetramethoxybenzene, 2-methoxyphenol, 1,3-dimethoxy-2-hydroxybenzene, 4-hydroxy-3,5-dimethoxybenzoic acid, 3-(4-hydroxy-3,5-dimethoxyphenyl)prop-2-enal, 4-hydroxy-3,5-dimethoxybenzaldehyde, 4-hydroxy-3,5-dimethoxyacetophenone, 3-(4-hydroxy-3,5-dimethoxyphenylprop-2-enoic acid, 4-enyl-2,6-dimethoxyphenol, 4-hydroxy-3-methoxybenzaldehyde, 3-hydroxy-4-methoxybenzaldehyde, 2-hydroxy-3-methoxybenzaldehyde, 2-hydroxy-5-methoxybenzaldehyde, 2-hydroxy-4-methoxybenzaldehyde, 3,4-dihydroxybenzaldehyde, 1-methoxy-4-[(E)-prop-1-en-yl]benzene, 1-bromo-4-methoxybenzene, 2-tert-butyl-4-methoxyphenol, ethoxybenzene, 1,3,5-trichloro-2-methoxybenzene, 1,3,5-tribromo-2-methoxybenzene, 4-(prop-2-en-1-yl)phenol, 1-methoxy-4(prop-2-en-1-yl)benzene, 5-(prop-2-en-1-yl)-2H-1,3-benzodioxole, 4-methoxy-2-[(E)-prop-1-yl]phenol, and 2-methoxy-4-(prop-1-en-yl)phenol.

In addition, the present disclosure provides a composition for depolymerizing an ester functional group-bearing polymer, the composition including: (A) at least one polyhydric alcohol, (B) a transesterification catalyst, and (C) a compound represented by Chemical Formula 1.

The transesterification catalyst may be an organic compound catalyst free of a metal or at least one selected from metal salts of groups 1A, 2A, and 2B.

The composition may include the polyhydric alcohol in an amount of 1 to 50 moles per mole of the repeating unit of the polymer to be depolymerized, the transesterification catalyst in an amount of 0.001 to 1 mole per mole of the repeating unit of the polymer to be depolymerized, and the compound represented by Chemical Formula 1 in an amount of 0.001 to 50 moles per mole of the repeating unit of the polymer to be depolymerized.

Also, the present disclosure provides a method for purifying a glycol-added monomer obtained by the depolymerization method of the present disclosure.

The purification method includes the steps of: (1) separating the compound represented by Chemical Formula 1 from the reaction mixture of depolymerization through gas-liquid separation or liquid-liquid extraction; (2) separating a solid containing the ester functional group-bearing polymer remaining reacted after step (1); (3) recovering the glycol-added monomer through recrystallization after the solid separating step (2).

In the purification method according to an embodiment of the present disclosure, the solid including the unreacted ester functional group-bearing polymer may be separated by physical filtration and a step of separating an oligomer may be carried out prior to the recrystallization of the glycol-added monomer in step (3).

Furthermore, the present disclosure provides an extractant for removing a color-expressing foreign matter from a monomer of an ester functional group-bearing polymer resin, the extractant including a compound represented by Chemical Formula 1.

In an embodiment of the present disclosure, the monomer of an ester functional group-bearing polymer resin may be obtained by depolymerization of the ester functional group-bearing polymer resin.

Moreover, the present disclosure provides a purification method for production of a monomer with high purity by removing foreign matter from a monomer of an ester functional group-bearing polymer resin, the method including the steps of: 1) adding a compound represented by Chemical Formula 1 to a monomer of an ester functional group-bearing polymer with a color-expressing foreign matter incorporated thereto and mixing same; 2) leaving the mixture of step 1) to induce liquid-liquid phase separation; and 3) separately discharging the upper organic solution phase and the lower aqueous solution phase in the phase-separated liquid-liquid layers, whereby color-expressing foreign matter can be removed from the monomer of the ester functional group-bearing polymer to afford a monomer in high purity.

In some embodiments of the purification method of the present disclosure, step 1) may include further adding water or a hydrophilic liquid immiscible with the compound represented by Chemical Formula 1 to the monomer having a

color-expressing foreign matter incorporated thereinto.

In some embodiments of the purification method of the present disclosure, the monomer is a reaction product from depolymerization of the ester functional group-bearing polymer or is obtained by partially purifying a mixture from resulting the depolymerization.

According to some embodiments of the present disclosure, the purification method may further include a step of carrying one or more rounds of further adding the compound represented by Chemical Formula 1 to the separated aqueous solution layer to extract the color-expressing foreign matter and a step of adding water or a hydrophilic liquid immiscible with the compound represented by Chemical Formula 1 to the separated organic solution layer and mixing same to perform phase separation again.

The purification method according to the present disclosure may further include a step of recovering the monomer of the ester functional group-bearing polymer resin from the separated aqueous solution phase through recrystallization.

In addition, the present disclosure provides a purification method for preparing a monomer in high purity, the method including the steps of: (a) adding a compound of Chemical Formula 1 to an ester functional group-bearing colored polymer; (b) depolymerizing the ester functional group-bearing polymer; (c) adding and mixing the reaction mixture obtained by the depolymerization with water or a hydrophilic solution immiscible with the compound represented by Chemical Formula 1; (d) leaving the mixture of step (c) to induce liquid-liquid phase separation; and (e) separately discharging the upper organic solution layer and the lower aqueous solution layer in the phase-separated liquid-liquid layers, whereby color-expressing foreign matter is removed from the monomer obtained by depolymerization of the ester functional group-bearing colored polymer.

In other embodiments of the present disclosure, the purification method may further include a step of recovering and recycling the organic solvent from the separately discharged organic solution layer in step (e) through distillation.

**Advantageous Effects**

The method for depolymerizing an ester functional group-bearing polymer according to the present disclosure enables a glycol-added monomer (e.g., bis(2-hydroxyethyl)terephthalate)), which has been a raw material before preparation of the polymer or is used for polymer resynthesis, to be produced in high yield and high purity and can find advantageous applications in mass production of regenerated monomers such as bis(2-hydroxyethyl)terephthalate (BHET) in high purity while polymers discarded after use, such as poly(ethylene terephthalate) (PET) or waste polyester fibers, serving as raw materials.

According to the present disclosure, milder reaction conditions than conventional reaction conditions and a reaction system composed of eco-friendly materials are applied to glycolysis-based decomposition to guarantee both reactivity for the decomposition of ester functional group-bearing polymers and selectivity for desired monomers, and after reaction, most of the materials including the catalyst can also be easily recovered by effectively applying thermodynamic phase separations such as liquid-liquid extraction or gas-liquid separation thereto, thus greatly reducing the content of impurities in the depolymerized product.

In addition, the present disclosure can acquire glycol-added monomers in higher yield and higher purity even at low temperatures and atmospheric pressure, compared to the glycolysis processes reported in previous documents.

Furthermore, the catalyst available in the technique according to the present disclosure can be selected from a broad spectrum of sources and is lower in toxicity and more inexpensive than conventional commercial catalysts (e.g., zinc acetate). Thus, the products thus achieved are harmless to the man body was well as being eco-friendly and economically beneficial.

Even when reused as raw materials for plastics or resin products, the depolymerized monomers according to the present disclosure can achieve a perfect chemical recycling technique, with no limitations imparted to the quality and use thereof.

According to the extractant and purification method of the present disclosure, it is possible to purify a monomer of an ester functional group-bearing polymer in high purity.

The monomer of the polymer is obtained by depolymerizing an ester functional group-bearing colored polymer and can be produced as a depolymerization monomer or addition monomer (e.g., BHET) in high yield and high purity with the equivalent quality to that of the monomer before use in polymer preparation.

Since the extractant does not participate in the depolymerization, it can be directly used in the monomer purification process, without separation after depolymerization and can be added before or after depolymerization, and thus, guarantees high process effectiveness and operation convenience.

Moreover, the extractant controls non-covalent interactions such as hydrogen bonds between foreign matter and additives, $\pi$-overlaps between aromatic rings in heterogenous compounds, etc., thereby decreasing the affinity for depolymerized low-molecular compounds. The isolated colored compounds are contained in the organic phase while the product terephthalate compound is distributed at a high concentration in the hydrophilic lower phase. Thus, physical phase

separation allows for the purification of the product in high purity. The post-depolymerization reaction processes themselves can be converted to purification processes or implement serial processes through continuous flow, so that the process flow can be simplified, with a reduction in energy consumption.

In the present disclosure, an operation condition milder than conventional separation processes can be applied and energy loss can be minimized. Also, eco-friendly compounds are applied to respective processes and the extract and part or entirety of the compounds used in the reaction can be recovered and reused through an additional separation process, whereby eco-friendly and economical purification processes can be established.

Also, the extractant and the purification method of the present disclosure do not require any complicated process units, but employ simple and repetitive phase separation processes to increase the purify of the depolymerization product, and thus can be applied to a technique for purification in high purity and high yield through various modes and methods.

## Brief Description of the Drawings

FIG. 1 is a $^1$H-NMR spectrum of BHETobtained by PET depolymerization according to a comparative embodiment of the present disclosure.

FIG. 2 is a $^1$H-NMR spectrum of a sample obtained through many rounds of water washes from the BHET prepared by PET depolymerization according to a comparative embodiment of the present disclosure.

FIG. 3 is a $^1$H-NMR spectrum of BHETobtained by PET depolymerization according to an embodiment of the present disclosure.

FIG. 4 is a liquid-liquid phase equilibrium plot of a binary compound composed of anisole, which is one of the extractants according to the present disclosure, and a hydrophilic solvent (water or ethylene glycol).

FIG. 5 shows $^1$H-NMR spectra for an organic dye extracted during depolymerization of colored waste PET polymers and purification of the depolymerized product and for purified depolymerized monomer products according to some comparative embodiments and embodiments of the present disclosure.

FIG. 6 shows $^1$H-NMR for an organic dye extracted during depolymerization of colored polyester fibers and purification of the depolymerized product and for purified depolymerized monomer products according to some embodiments of the present disclosure.

## Best Mode for Carrying out the Invention

Unless otherwise defined, all technical and scientific terms used herein have the same meanings as those typically understood by those skilled in the art to which the present disclosure belongs. In general, the nomenclature used herein is well known in the art and is typical.

Throughout the specification, when a portion may "include" a certain constituent element, unless explicitly described to the contrary, it may not be construed to exclude another constituent element but may be construed to further include other constituent elements.

The present disclosure provides a method for depolymerization of an ester functional group-bearing polymer, wherein a mixture containing (A) at least one polyhydric alcohol; (B) a transesterification catalyst; (C) a compound represented by Chemical Formula 1, below is brought into contact with the ester functional group-bearing polymer.

In addition, the present disclosure provides a composition for depolymerization of an ester functional group-bearing polymer, the composition including: (A) at least one polyhydric alcohol, (B) a transesterification catalyst, and (C) a compound represented by Chemical Formula 1, below.

In the present disclosure, at least one polyhydric alcohol is used as a raw material for a glycolysis reaction. The reaction catalyst may be a conventional catalyst such as acetate zinc, but may be a salt composed of a metal cation of group 1A, 2A, or 2B and an organic anion, such as carbonate, bicarbonate, alkoxide, or acetate, as a counter ion. Also, a non-metallic organic compound may be used as a catalyst.

In addition, the method of the present disclosure can greatly reduce activation energy in the decomposition for converting a functional group-bearing polymer raw material into glycol-added monomers by using the compound represented by Chemical Formula 1 as an additive. As stated in the foregoing, since low-cost catalysts containing various types of metals, such as group 2B, 1A, or 2A, or organic catalysts that catalyze decomposition at high temperatures can be selected as reaction materials, it is easy to secure economic feasibility or to control impurities by reusing and recovering materials. Accordingly, the present disclosure is characterized in that the reactivity of depolymerization can be maintained at a low temperature, and the purity, yield, and reaction selectivity of the depolymerized monomers are improved.

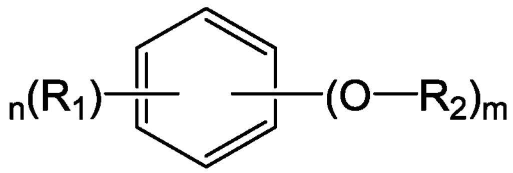

[Chemical Formula 1]

wherein,

- $R_1$ is any one selected from the group consisting of a hydrogen atom, a hydroxy, an aldehyde, a carboxyl, an alkyl of $C_1$-$C_6$, a cycloalkyl of $C_4$-$C_6$, and an aryl of $C_6$-$C_{12}$;
- n is an integer of 0 to 5 wherein when n is 2 or more, the corresponding $R_1$'s are same or different;
- $R_2$ is an alkyl of $C_1$-$C_{10}$; and
- m is an integer of 1 to 6 wherein when m is 2 or more, the corresponding -O-$R_2$'s are same or different.

The depolymerization method of the present disclosure is useful for depolymerizing an ester functional group-bearing polymer, wherein the ester functional group-bearing polymer may be in a form of single or mixed waste plastics, for example, may be in mixture with polyethylene, high-density polyethylene, low-density polyethylene, polypropylene, or a combination thereof. The enumerated polymers which may be in mixture with the ester functional group-bearing polymer are merely illustrative, but not limitative.

In addition, the ester functional group-bearing polymer may be a polymer formed through condensation polynmerization of a dicarboxylic acid and a dialcohol wherein the dicarboxylic acid is selected from the group consisting of terephthalic acid, naphthalene dicarboxylic acid, diphenyldicarboxylic acid, diphenylether dicarboxylic acid, diphenylsulfone dicarboxylic acid, diphenoxyethane dicarboxylic acid, succinic acid, adipic acid, sebacic acid, azelaic acid, decane dicarboxylic acid, cyclohexane dicarboxylic acid, trimellitic acid, pyromellitic acid, and a combination thereof and the dialcohol is selected from the group consisting of ethylene glycol, trimethylene glycol, 1,2-propanediol, tetramethylene glycol, neopentyl glycol, hexamethylene glycol, decamethylene glycol, dodecamethylene glycol, 1,4-cyclohexanediethanol, diethylene glycol, triethylene glycol, tetraethylene glycol, polyethylene glycol, dipropylene glycol, tripropylene glycol, tetrapropylene glycol, polypropylene glycol, di(tetramethylene) glycol, tri(tetramethylene) glycol, polytetramethylene glycol, pentaerythritol, 2,2-bis(4-β-hydroxyethoxyphenyl)propane, and a combination thereof.

By way of example, the ester functional group-bearing polymer may be selected from among polyethylene terephthalate (PET), polypropyleneterephthalate(PPT), polyglycolide or polyglycolic acid (PGA), polylactic acid (PLA), polycaprolactone (PCL), polyhydroxyalkanoate (PHA), polyhydroxybutyrate (PHB), polyethylene adipate (PEA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), Vectran, and a combination thereof.

The most common example of the ester functional group-bearing polymer is polyethylene terephthalate. In this regard, starting materials for use in preparing the polymer include terephthalic acid or derivative monomers thereof and ethylene glycol.

The ester functional group-bearing polymer used in the present disclosure may be in a pure state or in a mixed state containing various impurities. For instance, the starting material for the depolymerization of the present disclosure may contain bottle caps, adhesives, papers, residual liquid, dust, or a combination thereof in addition to the ester functional group-bearing polymer, but other remnant mixtures undefined above may be used.

In the method for depolymerizing an ester functional group-bearing polymer according to the present disclosure, the ester functional group-bearing polymer may be used in an amount of about 0.1-about 70% by weight, based on the total weight of the reaction raw materials. When the ester functional group-bearing polymer is initially fed in an amount less than 0.1 % by weight, no economic feasibility may be obtained. If the amount exceeds 70 % by weight, material transfer is limited, resulting in a remarkable decrease in reaction rate.

The polyhydric alcohol used for the glycolysis depolymerization of an ester functional group-bearing polymer is a compound bearing at least two alcohol functional groups and, more specifically, includes compounds with two hydroxy groups, like secondary linear alcohols, but is not limited thereto.

The polyhydric alcohol may be used in a range of 1 to 50 moles per mole of the repeat unit of the polymer.

Examples of the polyhydric alcohol bearing at least two alcohol functional groups include ethylene glycol, diethylene glycol, triethylene glycol, propylene glycol, dipropylene glycol, 1,3-butanediol, 1,4-butanediol, 3-methyl-1,5-pentanediol, 1,6-hexanediol, 1,7-octanediol, 1,9-nonanediol, neopentyl glycol, 1,4-cyclohexanediol, isosorbide, and 1,4-cyclohexane dimethanol.

In an embodiment of the present disclosure, the transesterification catalyst may be a metal-free organic catalyst. Non-limiting examples of the organic catalyst include linear amines (e.g., ethylene diamine, diethylenetriamine, tris(2-aminoethyl)amine, tetraethylenepentamine), amides (e.g., acetamide), guanidines (e.g., triazabicyclodecene), imidazoles (e.g., 2-ethyl-4-methylimidazole), ureas (e.g., urea, 1,1-dimethylurea, 1,3-dimethylurea), aromatic amines (e.g., benzenedimethanamine), and cycloalkyl amines (e.g., cyclohexylmethyl amine, dimethylcyclohexyl amine), and it is preferred that the catalytic compounds themselves do not directly participate in the reaction

Another example of the catalyst according to the present disclosure may be a salt composed of a metal cation of group 1A, 2A, or 2B and a counter anion selected from among carbonate, bicarbonate, alkoxide, and acetate.

In the catalyst, the metallic cation may be lithium ($Li^+$), sodium ($Na^+$), or potassium ($K^+$) in group 1A, magnesium ($Mg^{2+}$) or calcium ($Ca^{2+}$) in group 2A, or zinc ($Zn^{2+}$) in group 2B, but is not limited thereto.

The catalyst may be used in an amount of 0.001 to 1 mole and preferably in an amount of 0.01 to 0.1 mole per mole of repeat unit of the polymer.

The compound represented by Chemical Formula 1 is used as an additive for increasing activity of depolymerization of the ester functional group-bearing polymer and the selectivity for useful products. For instance, the substituent $R_2$ may be an alkyl of $C_1$-$C_{10}$, preferably an alkyl of $C_1$-$C_6$, and more preferably an alkyl of $C_1$-$C_3$.

In an embodiment of the present disclosure, the compound of Chemical Formula 1 contained in the additive may be at least one naturally occurring compound or synthetic compound selected from the group consisting of methoxybenzene, 1,2-dimethoxybenzene, 1,3-dimethoxybenzene, 1,4-dimethoxybenzene, 1,2,3-trimethoxybenzene, 1,2,4-trimethoxybenzene, 1,3,5-trimethoxybenzene, 1,2,3,4-tetramethoxybenzene, 1,2,3,5-tetramethoxybenzene, 1,2,4,5-tetramethoxybenzene, 2-methoxyphenol, 1,3-dimethoxy-2-hydroxybenzene, 4-hydroxy-3,5-dimethoxybenzoic acid, 3-(4-hydroxy-3,5-dimethoxyphenyl)prop-2-enal, 4-hydroxy-3,5-dimethoxybenzaldehyde, 4-hydroxy-3,5-dimethoxyacetophenone, 3-(4-hydroxy-3,5-dimethoxyphenylprop-2-enoic acid, 4-enyl-2,6-dimethoxyphenol, 4-hydroxy-3-methoxybenzaldehyde, 3-hydroxy-4-methoxybenzaldehyde, 2-hydroxy-3-methoxybenzaldehyde, 2-hydroxy-5-methoxybenzaldehyde, 2-hydroxy-4-methoxybenzaldehyde, 3,4-dihydroxybenzaldehyde, 1-methoxy-4-[(E)-prop-1-en-yl]benzene, 1-bromo-4-methoxybenzene, 2-tert-butyl-4-methoxyphenol, ethoxybenzene, 1,3,5-trichloro-2-methoxybenzene, 1,3,5-tribromo-2-methoxybenzene, 4-(prop-2-en-1-yl)phenol, 1-methoxy-4(prop-2-en-1-yl)benzene, 5-(prop-2-en-1-yl)-2H-1,3-benzodioxole, 4-methoxy-2-[(E)-prop-1-yl]phenol, and 2-methoxy-4-(prop-1-en-yl)phenol.

The additive may be used in an amount of 0.001 to 50 moles and preferably in an amount of 0.1 to 10 moles per mole of repeat unit of the polymer.

The method for depolymerizing a polymer according to the present disclosure provides a method for quickly depolymerizing an ester functional group-bearing polymer at a temperature of 100°C to 200°C and preferably at a temperature of 130°C to 160°C. As a result, the method for depolymerization of an ester functional group-bearing polymer, provided herein, can save energy because the reaction can proceed at a lower temperature than the conventional method. The method of the present disclosure is also economically feasible because the catalyst and additive used can be recovered and reused through an additional recovery process.

Furthermore, the depolymerization method for a polymer of the present disclosure may be carried out under the atmospheric pressure, but at an increased pressure depending on the formation of a vapor pressure of the additive or reactants used.

The depolymerization in the method disclosed herein may vary in reaction time, depending on the amount of the polymer used, but may be carried out for 0.5 hours to 12 hours, and preferably for 0.5 hours to 4 hours to minimize the yield of byproducts.

After the depolymerization reaction, a process of separating and removing unreacted polymeric materials by filtration from the reaction mixture may be further carried out.

The depolymerization of an ester functional group-bearing polymer according to the present disclosure uses a polyhydric alcohol and is thus accounted for by a type of glycolysis depolymerization.

The method for purifying the glycol-added monomers obtained according to the depolymerization method of the present disclosure may include: (1) a step of separating the additive compound represented by Chemical Formula 1 from the depolymerization reaction mixture through gas-liquid separation or liquid-liquid extraction; (2) a solid isolation step of isolating a solid including unreacted ester functional group-bearing polymer after step (1); and (3) a recovery step of recovering glycol-added monomers by recrystallization after the solid isolation of step (2).

The gas-liquid separation of the additive may be conducted separately from the reaction or continuously after the reaction is completed. For example, using a pre-installed low-temperature condenser, vaporized additives are condensed and refluxed into the reactor. Under this condition, the temperature is increased or maintained after completion of the reaction while the flow path of the condensed and refluxed additives is changed from the inside of the reactor to the outside to separate the additives.

Liquid-liquid extraction is a separation method in which water is added for recovery and purification of a target product. When water is added, a phase separation boundary is clearly formed and most additives can be physically separated by separating only the organic layer at a low temperature. Thereafter, most of the additives that remain in the aqueous phase can be separated and recovered by increasing the water content or lowering the temperature since they has very limited solubility in the aqueous phase. Crystallization allows for the recovery of most of the product. A trace amount of the additive that may remain in the purified target product may be perfectly removed by heating and drying at

50-150°C and preferably in a vacuum.

Gas-liquid phase separation for recovery of the additive according to the method disclosed herein includes a method in which the additive is separated into the outside by distillation in an additional step of increasing the temperature of the reactor to higher than the reaction temperature so as to vaporize or evaporate part or entirety of the additive. It may be the most economical to use water for the separation of additives through liquid-liquid extraction, but a hydrophilic material capable of inducing a thermodynamically unstable phase with the additive may be used. In this regard, the hydrophilic material may be glycols or diols used as a reactant.

The solid including the unreacted ester functional group-bearing polymer may be removed through various physical means such as precipitation, filtration, aggregation, suspension, compression, etc. after or before completion of the reaction, and can be fed again to the depolymerization. For a removal method using filtration, a filter having micropores with a subparticle size of the unreacted polymer may be used, and the method may be conducted in combination with pressurization or decompression to increase the flow rate of the filtrate.

The method for recovering the glycol-added monomer through a recrystallization process is more preferably performed after removal of the unreacted materials after the reaction. Water heated to more than room temperature, for example, a range of 60°C to 120°C and preferably a range of 90°C to 110°C or a solvent necessary for crystallization of the target product may be used, along with an additional step of separating oligomers through filtration. The filtrate may be maintained at a low temperature and recovered as a product by recrystallization. To control the concentration of impurities in the product, the physical separation and recrystallization process may be conducted repetitively. A step of separating oligomers may be carried out prior to the recrystallization of the glycol-added monomer in step (3).

In configuring a reaction system according to the depolymerization method of the present disclosure, a non-metallic catalyst may be used or a metal salt composed of a metal cation of group 1A or 2A. Alternatively, an organic counter anion such as carbonate, bicarbonate, alkoxide, or acetate may be used as a catalyst. In this case, the metallic ingredient that may remain in the purified monomer product is low in toxicity and can be removed in high yield during the purification process, making it easy to manage impurity concentration.

The present disclosure provides an extractant for removing a color-expressing impurity such as a pigment or a dye from a monomer of the ester functional group-bearing polymer, wherein the extractant includes a compound represented by Chemical Formula 1. In the compound represented by Chemical Formula 1, at least one alkyl group is linked to the aromatic ring moiety via a linker that bears at least one oxygen atom.

Examples of the compound represented by Chemical Formula 1 as the extractant are as enumerated for the compound of Chemical Formula 1 included in the additive for removing foreign matter, above, and the common content therebetween is omitted to avoid the undue complexity of the specification.

In an embodiment of the present disclosure, the monomer of the ester functional group-bearing polymer may be obtained as a result of depolymerizing the ester functional group-bearing polymer. In particular, the ester functional group-bearing polymer may be a colored polymer. The compound represented by Chemical Formula 1 does not directly participate as a reactant in the depolymerization of the ester functional group-bearing polymer, but retains the performance associated with depolymerization reactivity and product selectivity even in a low-temperature reaction and thus can be used in mixture of the polymer before the depolymerization.

The extractant according to the present disclosure is useful for removing color-expressing foreign matter such as pigments, dyes, etc. from the monomer of the ester functional group-bearing polymer. In this regard, the ester functional group-bearing polymer may be in a form of single or mixed waste plastics, for example, may be in mixture with polyethylene, high-density polyethylene, low-density polyethylene, polypropylene, or a combination thereof. The enumerated polymers which may be in mixture with the ester functional group-bearing polymer are merely illustrative, but not limitative.

In addition, the present disclosure provides a purification method for producing a monomer in high purity by removing foreign matter from the monomer of the ester functional group-bearing polymer.

The purification method includes the steps of: 1) adding a compound represented by Chemical Formula 1 to a monomer of an ester functional group-bearing polymer with a color-expressing foreign matter incorporated thereto and mixing same; 2) leaving the mixture of step 1) to induce liquid-liquid phase separation; and 3) separately discharging the upper organic solution phase and the lower aqueous solution phase in the phase-separated liquid-liquid layers.

In step 1), an extractant for removing foreign matter from the monomer of the ester functional group-bearing polymer is added and mixed to distribute more foreign matter in the extractant phase.

The temperature for extractive distribution in step 1) may be a general reaction temperature of depolymerization for the ester functional group-bearing polymer or a lower temperature. At the temperature, phase separation may be induced without adding a hydrophilic solvent, followed by an extraction process. However, when added, an excess of water, which is extremely dissoluble for the compound represented by Chemical Formula 1 makes a clearer phase separation boundary and induce an extremely asymmetrical distribution of depolymerization product and foreign matter, thereby enabling recovery of the monomer in high purity.

Since the compound represented by Chemical Formula 1 is relatively highly affinitive for color-expressing

organic materials such as pigments or dyes, the color-expressing foreign matter such as pigments or dyes migrates, for the most part, into an organic phase while most of the monomer products may be transferred, along with other hydrophobic compounds, into the hydrophilic phase.

The extractive distribution in step 1) may be carried out in a temperature range of 25-150°C.

For the process of removing foreign substances such as dyes or pigments and purifying monomers through liquid-liquid extraction according to the present disclosure, it may be most economically feasible to use water, but additives and hydrophilic substances that can induce thermodynamically unstable phases may be used. Alternatively, the process may be conducted in the presence of the polyhydric alcohol used as a reactant, without adding a separate solvent.

In step 2), the mixture that has undergone asymmetrical distribution of foreign matter is subjected to liquid-liquid phase separation.

The induction of the liquid-liquid phase separation may be performed continuously or batchwise using a known means such as a decanter, and a composition and temperature at which that thermodynamic miscibility is sufficiently low to allow phase separation to occur may be used. since the composition and temperature that allows for the phase separation occurs can be easily selected by a person skilled in the art, a detailed description thereof is be omitted.

As an example, FIG. 4 shows liquid-liquid phase equilibrium plots against temperature, established by titration, wherein reaction conditions and purification conditions for extraction according to an embodiment of the present disclosure are schematically depicted in order to distinguish the thermodynamic state of a binary compound composed of anisole, which is one of the extractants according to the present disclosure, and a hydrophilic solvent (water or ethylene glycol). Here, mole fractions of the organic extractant anisole are given on the x axis while reaction or purification temperatures are accounted for by the y axis. With reference to the spectrum of FIG. **1,** selection can be made of pertinent compositions and temperatures for phase separation.

In step 3), the organic phase (organic solution layer) and the aqueous (aqueous solution layer) are separately discharged from the mixture that has undergone phase separation. The separation may be achieved using a typical means and the organic and aqueous phases may be individually further processed to increase the recovery rate of the target product.

In some embodiments of the present disclosure, the purification method may further include a step of performing back extraction by additionally adding a hydrophilic solvent such as water to the organic solution layer (or organic phase) composed mostly of the primarily separated compound represented by Chemical Formula 1 to redistribute the monomer remining in the organic phase and additionally recovering a monomer product therefrom.

In addition, distillation for gas-liquid separation may be used to recover additives represented by Chemical Formula 1 from the organic phase separated and discharged by the liquid-liquid extraction according to the present disclosure, with the concomitant separation of dyes and pigments.

Most of the monomer products concentrated in the separately discharged aqueous solution layer can be recovered through crystallization, and the trace amounts of additives that may remain in the purified target product can be easily and completely removed by heating and drying at 50 to 150°C preferably in a vacuum.

The purification method for monomers from the ester functional group-bearing polymer according to the present disclosure may further include processes of physically filtering out, separating, and removing some foreign matter or may be carried out in association with the processes.

In an embodiment of the present disclosure, the monomer of the ester functional group-bearing polymer in step 1) may be a reaction mixture obtained by depolymerizing the ester functional group-bearing polymer or a compound derived therefrom. The depolymerization reaction mixture may be in a state unpurified after reaction or in a partially purified state.

When the monomer of ester functional group-bearing polymer in step 1) is a reaction mixture obtained by depolymerizing the ester functional group-bearing polymer or a compound derived therefrom, the extractant may be added before or after the depolymerization reaction process.

The ester functional group-bearing polymer may be decomposed to low-molecular weight compounds such as monomers, dimers, or oligomers through the depolymerization which may take advantage of hydrolysis, glycolysis, methanolysis, ammonolysis, or a combination thereof as a reaction route.

In detail, the method for purifying monomers of an ester functional group-bearing colored polymer in high purity by adding an extractant before a depolymerization reaction to remove a color-expressing foreign matter from a reaction mixture obtained by depolymerization may include the steps of: (a) adding a compound of Chemical Formula 1 to an ester functional group-bearing colored polymer; (b) depolymerizing the ester functional group-bearing polymer; (c) adding and mixing a reaction mixture obtained by the depolymerization with water or a hydrophilic solvent immiscible with the compound of Chemical Formula 1; (d) leading the mixture in step (c) to liquid-liquid phase separation; and (e) separately discharging the upper organic solution phase and the lower aqueous solution layer in the phase-separated liquid-liquid layers.

The purification method for monomers from the ester functional group-bearing polymer according to the present disclosure may further include processes of physically filtering out separating, and removing unreacted polymer materials from the depolymerization reaction mixture or may be carried out in association with the processes.

The depolymerization of the ester functional group-bearing polymer according to the present disclosure may be performed through a transesterification pathway using an alcohol, water, or a hydroxide in the presence or absence of a catalyst.

A better understanding of the present disclosure may be obtained through the following examples which are set forth to illustrate, but are not to be construed as limiting the present disclosure.

**1. Method for Depolymerization of Ester Functional Group-Bearing Polymer**

**[Raw materials 1 to 4]**

As ester functional group-bearing polymer materials, PET bottles discarded after use were cut into monolayer plastic flakes having an area of 1 $cm^2$ or less and a thickness of 0.3 mm or less (raw material 1)and plastic flakes having an area of 1 $cm^2$ or less and a thickness of 0.8 to 1.2 mm (composed of 10 or more layers) (raw material 2). Colored labels used in PET bottles discarded after use were cut into plastic flakes having an area of 1 $cm^2$ or less and a thickness of 0.1 mm or less (raw material 3). Polyester clothes discarded after use were cut into waster fiber flakes having an area of 1 $cm^2$ or less and a thickness of 1 mm or less (raw material 4).

**[EXPERIMENTAL EXAMPLE 1: Depolymerization Reaction Step (no additive added before reaction)]**

The polymer raw material prepared according to raw material 1 weighed about 10 g. Ethylene glycol, which is a dihydric alcohol polar solvent, was fed in an amount of about 38.75 g (12 moles per mole of the polymer repeating unit) into a reflux condenser-installed, 3-neck flask and heated and stirred using a heating-type magnetic stirrer. When the temperature reached 177°C, 10 g of the polymer raw material was fed, followed by temperature elevation together with stirring. When the reaction mixture was heated to 197°C or the reflux temperature, the catalyst was added in an amount of 0.04 mols per mol of the polymer repeating unit to initiate the catalytic reaction. While continuously stirring for 2 hours, the reaction temperature was constantly maintained with a deviation of $\pm$0.5°C and the reaction was conducted using a condenser with one end exposed to atmospheric pressure. From the mixture obtained after the reaction, unreacted materials were separated and quantitated. Concentration of monomers, dimers, and byproducts such as mono (hydroxyethyl) terephthalate (MHET), oligomers, etc. were measured using high-performance liquid chromatography (HPLC, reverse phase C18 column (250 mm, 5 microns), UV detector ($\lambda$=254 nm)) calibrated with a standard sample. As the mobile phase for HPLC analysis, a mixed solution with a volume ratio of methanol: water of 70:30 was used while maintaining a constant flow rate of 0.7 mL/min.

**[EXPERIMENTAL EXAMPLE 2: Depolymerization Reaction Step (additive added before reaction)]**

Before starting a depolymerization reaction, an additive was fed in an amount of 4 moles per mole of the polymer repeating unit, together with about 38.75 g of the dihydric alcohol polar solvent ethylene glycol (12 moles per mole of the polymer repeating unit) into a reflux condenser-installed, 3-neck flask and heated and stirred using a heating-type magnetic stirrer under an atmospheric pressure. When the temperature reached 20°C lower than the final reaction temperature, 10 g of the polymer raw material was fed, followed by temperature elevation together with stirring. When the reaction mixture was heated to the reaction temperature, the catalyst was added in an amount of 0.04 mols per mol of the polymer repeating unit to initiate the catalytic reaction. The reaction and analysis were carried out in the same manner as in Experimental Example 1, with the exception that the additive was additionally added and the reaction temperature was maintained at 153°C or lower.

**[EXPERIMENTAL EXAMPLE 3: Purification of Product]**

In a 500-mL round flask, a mixture of the reaction product and 400 mL distilled water were continuously stirred for about 1 hour within the range of 90 to 95 °C. After the reaction, oligomers in the mixture were removed through filter paper (Whatman, pore diameter: 3 microns) placed in a filter by a vacuum pump. The filtrate obtained primarily was filtered again through the same filter to separate and remove recrystallized oligomers and solids. The resulting filtrate was crystallized by storage for at least 16 hours in a low-temperature bath maintained at 4°C or less, and the precipitates were collected through a vacuum filter and dried for 6 hours at about 80°C in an oven to afford a glycol-added product as a white powder. It was quantified.

The purified product was examined for the content of metals remaining therein. To this end, about 0.1 g of the product was dissolved and pre-treated with acid, followed by analysis by inductively coupled plasma-atomic emission spectrometry (ICP-AES, Model: Thermo Fisher Scientific iCAP 6500Duo) and inductively coupled plasma - mass spectrometry (ICP-MS, Model: Thermo Fisher Scientific X Series).

For use in characterizing the purified product, a sample with a monomer concentration of 6.25% was prepared by dissolving about 0.1 g of the purified product in a DMSO-$d_6$ solvent. NMR spectra were obtained by $^1$H nuclear magnetic resonance ($^1$H-NMR, Model: Bruker AVANCE ‖$^+$ 500 MHz).

**[EXPERIMENTAL EXAMPLE 4: Additional Purification Step]**

The white powder obtained in Experimental Example 3 was added to a 250-mL round flask containing 100 mL of distilled water and stirred for 1 hour while maintaining the temperature in the range of 90 to 95°C. Then, the mixture was transferred to a low-temperature bath maintained at 4°C or less, and left to stand for 16 hours or more to perform recrystallization. The same filter as in Experimental Example 3 was used to recover additionally washed precipitates. These precipitates were dried for 6 hours in an oven maintained at about 80°C to afford a glycol-added monomer as a white powder. The powder product obtained through the additional purification was subjected to ICP-AES, ICP-MS, and $^1$H-NMR analysis in the same manner as in Experimental Example 3.

**[Calculation Formula]**

Among the products obtained from the depolymerization performed according to Comparative Examples and Examples of the present disclosure, unreacted substances in the solid phase and the products dissolved as a liquid phase were quantified by the gravimetric method and through dilution and HPLC analysis, respectively. From the data, the conversion rate and the yield of the product were calculated according to the following equations:

Polymer conversion rate by depolymerization (%) = (initially fed polymer amount - unreacted polymer amount) / (initially fed polymer amount)×100

Monomer yield by depolymerization (%) = (mole of obtained BHET)/(mole of terephthalate unit in fed polymer structure)×100

Dimer yield by depolymerization (%) = (mole of obtained dimonomer)/( mole of terephthalate unit in fed polymer structure)×100

Byproduct (MHET) yield by depolymerization (%) = (mole of obtained MHET)/(mole of terephthalate unit in fed polymer structure)×100

**1.1 Comparison of Reaction Characteristics of Depolymerization Using Metal Salts as Catalysts**

TABLE 1

| Ex. # | Catalyst | Mole of Cpd. per mole of polymer (PET) repeating unit | | | Conversion rate (%) | Yield (%) | | |
|---|---|---|---|---|---|---|---|---|
| | | EG | Additive | Catalyst | | BHET | Dimer | MHET |
| C. Ex. 1 | Zn $(OAc)_2$ | 12 | 0 | 0.04 | 100.0 | 90.06 | 4.69 | 0.37 |
| C. Ex. 2 | KOAc | 12 | 0 | 0.04 | 100.0 | 83.52 | 4.06 | 2.36 |
| C. Ex. 3 | NaOAc | 12 | 0 | 0.04 | 100.0 | 84.46 | 4.29 | 3.92 |

Reaction condition: (fed polymer) raw material 1, (rxn. temp.) 197°C, (rxn. time) 2h

EG: ethylene glycol, BHET: bis(2-hydroxyethyl) terephthalate,
Dimer: dimer of BHET, MHET: mono (hydroxyethyl) terephthalate,
Zn(OAc)2: zinc acetate, NaOAc: sodium acetate, KOAc : potassium acetate

Summarized in Table 1 are the data that were obtained by evaluating the performance of the glycolysis reaction of the ester functional group-bearing polymer raw material under the most commonly used reaction conditions to compare and explain the effects of the Examples in the present disclosure. To this end, an excess of ethylene glycol (12 moles per

mole of polymer repeating unit) was used and the glycolysis reaction was performed at atmospheric pressure and under reflux conditions of ethylene glycol (197°C). Depolymerization of polyethylene terephthalate was performed according to the method described in Experimental Example 1 in the presence of zinc acetate catalyst, which is most commonly used for the reaction, and the data is shown for Comparative Example 1. Depolymerization was performed in the same manner with exception that alkaline acetates were used as modified and combined catalysts in the same moles, instead of zinc acetate for Comparative Example 1, and the data are given for Comparative Examples 2 and 3.

As previously reported in a number of patent and non-patent prior literature, when zinc acetate was applied as a catalyst (Comparative Example 1), the polymer was completely decomposed in just two hours of reaction, and BHET was obtained in a high yield of 90%. Even when the depolymerization of polyethylene terephthalate was performed under the same reaction conditions, except that acetate having an alkali metal such as potassium or sodium as a cation was used as a catalyst, high reactivity was observed, but the selectivity for the monomer BHET was low. A relatively low yield of BHET may affect productivity, and may cause various economic and environmental problems, such as the burden of a purification process to separate low-value by-products and the treatment of by-products.

### 1.2 PET Glycolysis at Low Temperature Conditions

When a glycolysis reaction route is selected for depolymerization of an ester functional group-bearing polymer, as shown in the depolymerization reaction conditions of Comparative Examples 1 to 3 above, the reaction is commonly performed at a high temperature equal to or higher than the boiling point (197°C) of ethylene glycol added as an additional reactant of the transesterification reaction. Even if an excessive amount of catalyst is used or a very low concentration of the polymer raw material is applied to the reaction, a low reaction temperature prevents sufficient activation energy for the depolymerization reaction, making the reactivity very low or relatively increasing the rate of side reactions, with the consequent failure in obtaining the desired product. On the other hand, in the case of a manufacturing process requiring a high reaction temperature, limitations may be imparted to the application method of fuel or utilities for supplying a heat source for the reaction, and excessive costs may be required for investment in facilities and process operation. Therefore, designing a low-temperature glycolysis depolymerization reaction capable of guaranteeing high reactivity and high yield can provide an effective and decisive means for securing the economics of the process.

Most of the previous literatures on depolymerization of ester functional group-bearing polymers along the glycolysis reaction pathway suggest reaction conditions corresponding to the boiling point of ethyleneglycol (197°C) or higher, disclosing that it is very difficult to lower the reaction activation energy even in the presence of a catalyst composed of only single or specific components or a combination thereof. In particular, practical examples of a reaction system capable of significantly lowering the reaction temperature while maintaining equivalent performance have not been reported.

To overcome the problems, the present disclosure proposes a new reaction pathway through which ester functional group-bearing polymers can be depolymerized in a temperature range lower by at least 40°C than those for general glycolysis reactions by employing as an additive a compound bearing at least one oxygen atoms between an aromatic ring and an alkyl group to remarkably lower the activation energy of the catalytic rection. Suggested thus is a method for implementing a low-temperature glycolysis that employs as a catalyst an organic compound or a metal salt including metal cations of Group IA or 2A, which has difficulty in retaining performance or is difficult to recover because it is apt to degrade at a high temperature.

**To specifically explain the effect of the low-temperature glycolysis reaction according to the present disclosure, data of the glycolysis reaction conducted at a low temperature (153°C) condition with the same composition as the Comparative Examples are shown in Table 2. Characteristic of the low-temperature glycolysis reactions according to the present disclosure were elucidated. To this end, data for individual catalysts in the presence or absence of an additive were obtained for Comparative Examples and Examples under the same conditions.**

In Comparative Examples 4 to 6, the glycolysis depolymerization was conducted in the same manner for the polymer raw material, reaction catalyst, and ethylene glycol as in the previous Comparative Examples, with the exception that the reaction temperature was maintained at 153°C. A depolymerization rection was carried out with zinc acetate as a catalyst in Comparative Example 4 and potassium acetate and sodium acetate as catalysts in Comparative Examples 5 and 6, respectively. In Example 1, a depolymerization reaction was carried out using a zinc acetate catalyst and anisole corresponding to the compound of Chemical Formula 1 as an additive according to Experimental Example 2. In Examples 2 and 3, the same procedure as in Example 1 were carried out, with the exception of using potassium acetate and sodium acetate, respectively.

Anisole used as the additive is extracted as a natural product, but can also be industrially synthesized in large quantities. It is used as a raw material or precursor for the manufacture of aromatics or perfumes, and exhibits very low toxicity compared to other aromatic compounds.

Having an amorphous structure and a wide unit area with a thickness of 0.3 mm or less, the PET bottle-derived

raw materials fed in the reaction in the Comparative Examples and Examples above can maintain high effective contact areas with additives, catalysts, and reactants from the initial stage of depolymerization. In contrast, raw materials for depolymerization which are different in degree of polymerization of polymers, properties, and geometric shape such as material thickness, may affect mass transfer and reaction rate, which leads to a great difference in the yield and performance of the final product of depolymerization. In Examples 4 to 6, among the waste plastics that can be discharged in daily life, multilayered PET bottles, PET colored label paper, and polyester fibers were pulverized to prepare waste resins with an area of 1 $cm^2$ or less and these raw materials were subjected to depolymerization. The depolymerization was carried out in the same condition as in Example 3 using raw material 1.

TABLE 2

| Ex. # | Catalyst | Raw material # | Mole of Cpd. per mole of polymer (PET) repeating unit | | | Conversion rate (%) | Yield (%) | | |
|---|---|---|---|---|---|---|---|---|---|
| | | | EG | Additive | Catalyst | | BHET | Dimer | MHET |
| C. Ex. 4 | Zn (OAc) 2 | 1 | 12 | 0 | 0.04 | 9.41 | 3.53 | 0.06 | 0.11 |
| C. Ex. 5 | KOAC | 1 | 12 | 0 | 0.04 | 13.92 | 6.06 | 0.00 | 1.19 |
| C. Ex. 6 | NaOAc | 1 | 12 | 0 | 0.04 | 14.06 | 5.18 | 0.00 | 1.37 |
| Ex. 1 | Zn (OAc) 2 | 1 | 12 | 4 | 0.04 | 100.0 | 82.47 | 4.60 | 1.19 |
| Ex. 2 | KOAC | 1 | 12 | 4 | 0.04 | 100.0 | 86.50 | 4.89 | 1.77 |
| Ex. 3 | NaOAc | 1 | 12 | 4 | 0.04 | 100.0 | 86.91 | 4.98 | 1.82 |
| Ex. 4 | NaOAc | 2 | 12 | 4 | 0.04 | 99.53 | 82.50 | 4.43 | 1.78 |
| Ex. 5 | NaOAc | 3 | 12 | 4 | 0.04 | 100.0 | 77.63 | 1.87 | 2.01 |
| Ex. 6 | NaOAc | 4 | 12 | 4 | 0.04 | 100.0 | 88.33 | 5.26 | 1.03 |
| Rxn. condition: (Rxn. Temp.) 153°C, (additive) anisole, (Rxn. Time ) 2h EG: ethylene glycol, BHET: bis(2-hydroxyethyl) terephthalate, Dimer: dimer of BHET, MHET: mono(hydroxyethyl) terephthalate, $Zn(OAc)_2$: zinc acetate, NaOAc: sodium acetate, KOAc : potassium acetate | | | | | | | | | |

As shown in Table 1 above, most of the PET was decomposed in Comparative Example 1 where a zinc acetate catalyst and a high temperature of 197°C were applied. However, according to the results shown for Comparative Example 4 in Table 2, when depolymerization was performed at a low temperature (153°C) with the same reaction mixture composition, an extremely poor reactivity was observed, with a conversion rate less than 10%. In addition, a relatively high ratio of by-products to the desired product was obtained.

In Comparative Examples 5 and 6, the same procedure as in Comparative Example 4 was carried out, with the exception of using meal salts include alkali metal cations, in detail, potassium acetate and sodium acetate, instead of zinc acetate, as respective catalysts. Compared to Comparative Example 4, the conversion rate slightly increased, but the reactivity was not greatly improved. At the same time, the production of by-products was greatly increased, and higher BHET selectivity was observed upon using zinc acetate (Comparative Examples 1 and 4).

The reaction result of Example 1 was obtained by performing glycolysis at a low temperature (153°C) using the reactant composition of Comparative Example 4 plus anisole in the presence of zinc acetate. Comparing the depolymerization results according to Comparative Example 4 and Example 1, the additive according to the present disclosure was observed to have a very dramatic effect on the change in depolymerization performance. The depolymerization reaction in Example 1 obtained a BHET yield of 82.5% within 2 hours despite the reaction at a temperature of 40°C, and a BHET yield of 85% to 90% when it was continued for 3 hours or more.

A more dramatic effect was observed when the catalyst was changed to an alkali-based metal acetic acid catalyst. For example, the glycolysis reaction results obtained at a low temperature (153°C) in the presence of the additive with potassium acetate and sodium acetate serving as catalysts (Examples 2 and 3) were compared with those of high-temperature reactions (Comparative Examples 2 and 3) and low-temperature reactions with no additives (Comparative Examples 5 and 6). In Example 2 (or Example 3) where low-temperature glycolysis was carried out with the reactant composition of Comparative Example 5 (or Comparative Example 6) plus anisole, high depolymerization activity was observed as in Example 1 wherein depolymerization was carried out in the presence of an additive with zinc acetate serving as a catalyst. When depolymerization was performed a reaction system using an alkali metal as a catalyst plus anisole, high reactivity was observed as all raw material PET was completely decomposed within 2 hours despite maintaining the reaction temperature as low as 153°C. The selectivity was also greatly improved. The addition of anisole to

a catalyst system containing other modified catalysts effective for glycolysis at high temperature such as 197°C, that is, other type catalysts of alkaline metal-containing salts or non-metallic organic catalysts such as guanidines, dramatically enhanced the reactivity in the glycolysis at low temperatures such as 153°C. For some catalyst systems, selectivity was also enhanced. The low-temperature depolymerization performance according to the type of catalyst will be described in more detail in Section 1.7.

Comparing the depolymerization reaction results of Examples 4 to 6 using polymer raw materials having different properties under the same conditions, it was found that a higher yield of BHET was obtained in Example 6 using polyester fiber as a raw material, which is expected to have low mass transfer restrictions during the depolymerization process due to its low intrinsic viscosity (IV ~ 0.65) than in the case of using PET flakes as a raw material (IV=0.74). In Example 4 using multilayered PET flakes as a raw material, only 2-hour reaction resulted in a BHET yield of as low as 82.5%.

In Example 5 where a colored PET thin film, which is not expected to be significantly affected by mass transfer, was used as a raw material, the initial reactivity was observed high, but the yield remained only at 80% or less. In this case, the yield was not improved even after the mixture was left in the reaction conditions for a long time, and the low BHET yield might be attributed to the fact that impurities such as pigments partially adversely affected the depolymerization performance.

### 1.3 Temperature Range for Low-Temperature Depolymerization Reaction

The following Examples are set forth to explain a temperature range in which the reaction or depolymerization performance at low temperatures is effective while the aforementioned reactant compositions effective for the low-temperature glycolysis reaction are maintained. In order to determine qualitative and quantitative reaction characteristics of depolymerization, various temperature conditions are given, with the other conditions remaining unchanged.

TABLE 3

| Ex. # | Rxn. Temp. (°C) | Rxn. Time (h) | Conversion rate (%) | Yield (%) | | |
|---|---|---|---|---|---|---|
| | | | | BHET | Dimer | MHET |
| Ex. 7 | 130 | 3 | 17.50 | 9.70 | 0.06 | 0.60 |
| | | 6 | 33.68 | 23.79 | 0.35 | 1.36 |
| Ex. 8 | 135 | 3 | 25.10 | 20.96 | 0.38 | 0.88 |
| | | 6 | 53.03 | 43.04 | 1.32 | 1.81 |
| Ex. 9 | 140 | 3 | 50.82 | 42.18 | 1.33 | 1.35 |
| | | 6 | 91.85 | 72.22 | 3.71 | 2.19 |
| Ex. 10 | 145 | 3 | 75.44 | 63.50 | 3.08 | 1.64 |
| | | 6 | 99.72 | 79.51 | 4.46 | 2.59 |
| Ex. 11 | 150 | 3 | 100.0 | 84.10 | 4.84 | 2.10 |
| | | 6 | 100.0 | 84.10 | 4.83 | 3.23 |
| Rxn. condition: (fed polymer) raw material 1, (additive) anisole, (moles of compound per mole of polymer repeating unit) NaOAc 0.04 moles, anisole 4 moles, EG 12 moles, (Rxn. Time) 2h BHET: bis(2-hydroxyethyl) terephthalate, Dimer: dimer of BHET, MHET: mono(hydroxyethyl) terephthalate, NaOAc: sodium acetate, | | | | | | |

Glycolysis depolymerization was carried out in the same manner as in Example 3, except that the reaction temperature was maintained at 130 to 150°C, and the results are summarized in Table 3. Sodium acetate (NaOAc) as a catalyst was used in an amount of 0.04 moles, anisole in an amount of 4 moles, and ethylene glycol in an amount of 12 moles per mole of the polymer repeating unit. Experiments were repeated to conduct the reaction for a total of 3 hour or 6 hours. The products thus obtained were prepared into samples in the same manner, quantitatively analyzed by HPLC, and then examined for conversion rates and yields.

According to the reactant composition of the present disclosure, low-temperature glycolysis was performed on PET raw materials in the presence of anisole. As a result, clear and effective reaction characteristics were detected at 130°C or higher. When the raw material was subjected to the reaction for a long period of time (6 hours) at 145°C or higher, a very dramatic effect was observed, with a conversion rate close to 100%.

In Example 11 where depolymerization was performed at 150°C, the polymer was completely decomposed within 3 hours due to high reactivity, but upon exposure to long-term reaction conditions, a rather low selectivity towards the

monomer product (BHET) was observed. This is because the yield of MHET gradually increases due to the hydrolysis of BHET, which is a side reaction pathway, when the already produced target product is continuously exposed to high temperatures. Therefore, in order to completely decompose the mass of the polymer and prevent the side reaction from proceeding, the final yield of the desired product is preferably improved by optimizing not only the temperature but also the reaction time.

**1.4 Performance Evaluation of Depolymerization Reaction according to Amount of Additive**

The following Examples are set forth to explain an amount range of an additive in which the effect of the additive is exhibited while the aforementioned reactant compositions and reaction conditions effective for the low-temperature glycolysis reaction are maintained. In order to determine qualitative and quantitative reaction characteristics of depolymerization, the additive was used in various amounts, with the other conditions remaining unchanged.

Low-temperature (153°C) depolymerization was conducted for 2 hours without additives (Comparative Examples 5 and 6) or in the presence of various amounts of the additive (Examples 12 and 13), and distributions of the products thus obtained were measured. The measurements are summarized in Table 4. That is, depolymerization was conducted while the additive anisole was added in an amount of 0 g to 39.4 g (7 moles of per mole of the polymer repeating unit). Glycolysis depolymerization was carried out in the same manner as in Example 2 or 3, except for the amounts of the additive.

TABLE 4

| Catalyst type | Ex. # | Mole of Cpd. per mole of polymer (PET) repeating unit | | | Conversion rate (%) | Yield (%) | | |
|---|---|---|---|---|---|---|---|---|
| | | EG | Additive | Catalyst | | BHET | Dimer | MHET |
| KOAC | C. Ex. 5 | 12 | 0 | 0.04 | 13.92 | 6.06 | 0.00 | 1.19 |
| | Ex. 12 | 12 | 1 | 0.04 | 32.05 | 24.06 | 0.35 | 2.06 |
| | | 12 | 2 | 0.04 | 91.47 | 76.83 | 3.70 | 2.18 |
| | | 12 | 3 | 0.04 | 99.72 | 83.65 | 4.56 | 1.85 |
| | | 12 | 4 | 0.04 | 100.0 | 86.50 | 4.89 | 1.77 |
| | | 12 | 5 | 0.04 | 100.0 | 85.22 | 4.81 | 1.69 |
| | | 12 | 7 | 0.04 | 100.0 | 83.19 | 5.39 | 1.69 |
| NaOAc | C. Ex. 6 | 12 | 0 | 0.04 | 14.07 | 5.19 | 0.00 | 1.37 |
| | Ex. 13 | 12 | 1 | 0.04 | 33.44 | 26.30 | 0.41 | 1.05 |
| | | 12 | 2 | 0.04 | 75.49 | 63.85 | 2.50 | 1.20 |
| | | 12 | 3 | 0.04 | 99.73 | 84.30 | 4.39 | 1.11 |
| | | 12 | 4 | 0.04 | 100.0 | 86.91 | 4.98 | 1.82 |
| | | 12 | 5 | 0.04 | 100.0 | 85.96 | 4.58 | 0.92 |
| | | 12 | 7 | 0.04 | 100.0 | 82.55 | 4.68 | 0.85 |

Rxn. Condition: (fed polymer) raw material 1, (additive) anisole, (Rxn. Temp.) 153°C, (Rxn. Time) 2h
EG: ethylene glycol, BHET: bis(2-hydroxyethyl) terephthalate, Dimer: dimer of BHET, MHET: mono(hydroxyethyl) terephthalate, NaOAc: sodium acetate, KOAc : potassium acetate

Very poor glycolysis reaction performance was observed in Comparative Examples 5 and 6 where metal salts containing alkali metal cations were used as catalysts and no additives corresponding to Chemical Formula 1 according to the present disclosure was added. In contrast, from the results of Examples 12 and 13 in which even a small amount of anisole was added, it can be seen that not only the reactivity increased, but also the selectivity of BHET produced according to the glycolysis reaction of PET was greatly improved. In particular, when the amount of additives added to the applied polymer raw material increased to 3 molar ratio or more, it can be seen that most of the polymer was decomposed in just two hours of reaction and a high value was maintained for the selectivity of the target product.

When the temperature is lowered below 100°C, the additive used undergoes phase separation from excess ethylene glycol used as a reactant. In addition, even if an excess of water is added to purify the target product, only a very

limited concentration of the additive remains in the aqueous solution in which the target product is dissolved. Thus, the additive can be easily recovered. Hence, the additive can be easily separated by liquid-liquid extraction, but rather, a gas-liquid separation method may be more advantageous in terms of efficiency, wherein at the end of the reaction, the reaction temperature is raised to the boiling point of the additive, and then some or all of the condensed additives are separated through a condenser into an external flow. The additive according to the present disclosure is a compound that does not directly participate in the reaction, but has properties similar to catalysts because it has a clear effect of lowering the activation energy of the reaction. However, since the additive does not directly improve reactivity in the absence of metal salts or other catalysts, it acts as a co-catalyst.

### 1.5 Performance Evaluation of Depolymerization reaction according to Amount of Catalyst

The following Examples are set forth to explain the effect according to amounts of the catalysts under while maintaining the composition and reaction conditions of the reactants effective for the low-temperature glycolysis. In order to determine reaction characteristics of depolymerization, the catalysts were used in various amounts, with the other conditions remaining unchanged (reaction temperature: 153°C, reaction time: 2 hours, additive: anisole).

TABLE 5

| Ex. # | Catalyst type | Mole of Cpd. per mole of polymer (PET) repeating unit | | | Conversion rate (%) | Yield (%) | | |
|---|---|---|---|---|---|---|---|---|
| | | EG | Additive | Catalyst | | BHET | Dimer | MHET |
| Ex.14 | KOAC | 12 | 4 | 0.00 | 3.53 | 0.01 | 0.00 | 0.00 |
| | | 12 | 4 | 0.01 | 27.21 | 20.51 | 0.45 | 0.17 |
| | | 12 | 4 | 0.02 | 81.00 | 67.49 | 3.06 | 0.36 |
| | | 12 | 4 | 0.03 | 95.68 | 80.32 | 4.26 | 0.51 |
| | | 12 | 4 | 0.04 | 100.0 | 86.50 | 4.89 | 1.06 |
| | | 12 | 4 | 0.05 | 100.0 | 84.68 | 4.50 | 1.07 |
| | | 12 | 4 | 0.06 | 100.0 | 82.91 | 4.38 | 1.28 |
| | | 12 | 4 | 0.08 | 100.0 | 83.73 | 4.63 | 1.98 |
| | | 12 | 4 | 0.10 | 100.0 | 80.89 | 4.24 | 2.55 |
| | | 12 | 4 | 0.20 | 100.0 | 80.12 | 4.32 | 3.16 |
| Ex. 15 | NaOAc | 12 | 4 | 0.00 | 3.53 | 0.01 | 0.00 | 0.00 |
| | | 12 | 4 | 0.01 | 24.24 | 17.67 | 0.44 | 0.31 |
| | | 12 | 4 | 0.02 | 92.52 | 74.97 | 3.60 | 0.41 |
| | | 12 | 4 | 0.03 | 99.85 | 83.93 | 4.40 | 0.65 |
| | | 12 | 4 | 0.04 | 100.0 | 85.58 | 4.54 | 1.03 |
| | | 12 | 4 | 0.05 | 100.0 | 86.13 | 4.58 | 1.24 |
| | | 12 | 4 | 0.06 | 100.0 | 83.48 | 4.62 | 2.68 |
| | | 12 | 4 | 0.08 | 100.0 | 84.16 | 4.63 | 2.94 |
| | | 12 | 4 | 0.10 | 100.0 | 82.38 | 4.53 | 4.62 |
| | | 12 | 4 | 0.23 | 100.0 | 80.39 | 4.37 | 4.89 |
| Rxn. Condition: (fed polymer) raw material 1, (additive) anisole, (Rxn. Temp.) 153°C, (Rxn. Time) 2h<br>EG: ethylene glycol, BHET: bis(2-hydroxyethyl) terephthalate, Dimer: dimer of BHET, MHET: mono(hydroxyethyl) terephthalate, NaOAc: sodium acetate, KOAC : potassium acetate | | | | | | | | |

In Examples 14 and 15, glycolysis depolymerization was conducted and evaluated in the same procedures as in Examples 2 and 3, respectively, with the exception that the catalysts (metal salts containing alkaline metal cations) were fed in an amount of 0 g to 1.0 g (up to 0.20 moles per mole of the polymer repeating unit). The evaluation data thus obtained are summarized in Table 5.

When the addition amount of the catalyst was added in a small amount of 0.01 mol or less relative to the repeating unit of the polymer raw material, it was observed that the reactivity was significantly lowered, but the selectivity of the target product, BHET, was observed to be high. This seems to be because an increase in the amount of catalyst added has a greater effect on the increase in the side reaction rate. When the amount of the catalyst was added in excess of 0.01 mol relative to the repeating unit of the polymer raw material, most of the added PET was decomposed due to its high reactivity, and when the amount exceeded 0.03, complete decomposition occurred. When the catalysts were added in an amount of 0.03 moles to 0.10 mole (both exclusive) to cause complete decomposition, only a slight change was observed in the selectivity or yield for the desired product (BHET) depending on the amount of catalyst. However, when the amounts of the catalysts exceed 0.10 mol, the amount of by-product production increased, with the concomitant decrease of the BHET yield. As a result of observing the depolymerization reaction characteristics according to the input amount of catalyst, it is possible to maintain and control the reaction performance according to the amount of catalyst input in a relatively wide range. These data indicate that it is possible to secure flexibility during process operation because, even if the catalyst concentration is not precisely controlled in reusing some catalysts after the completion of the reaction, only some of the catalysts identified as lost need to be replenished.

### 1.6 Performance Evaluation of Depolymerization Reaction according to Types of Additives

The following Examples are set forth to explain the effect on the reaction when the additive according to the present disclosure was changed partially or totally while maintaining the composition and reaction conditions of the reactants effective for the low-temperature glycolysis. In order to determine reaction characteristics of depolymerization, depolymerization performance was observed as the additive that bears an oxygen atom as a linker between the aromatic ring moiety and an alkyl group was used in modified forms, such as a different number of the functional linkers, with the other conditions remaining unchanged (reaction temperature: 153°C, reaction time: 2 hours, catalyst: KOAc).

TABLE 6

| Ex. # | Catalyst type | Mole of Cpd. per mole of polymer (PET) repeating unit | | | Conversion rate (%) | Yield (%) | | |
|---|---|---|---|---|---|---|---|---|
| | | EG | Additive | Catalyst | | BHET | Dimer | MHET |
| C. Ex. 5 | - | 12 | 0 | 0.04 | 13.92 | 6.06 | 0.00 | 1.19 |
| Ex. 2 | Anisole | 12 | 4 | 0.04 | 100.00 | 86.50 | 4.89 | 1.77 |
| Ex. 16 | 1, 2-DMB | 12 | 4 | 0.04 | 69.76 | 58.38 | 2.19 | 1.43 |
| Ex. 17 | 1, 4-DMB | 12 | 4 | 0.04 | 70.18 | 57.35 | 2.62 | 1.56 |
| Ex. 18 | 1,3,5-TMB | 12 | 4 | 0.04 | 96.09 | 65.13 | 3.58 | 1.53 |
| Ex. 19 | Phenetole | 12 | 4 | 0.04 | 41.63 | 36.57 | 0.69 | 2.46 |
| Ex. 20 | Guaiacol | 12 | 4 | 0.04 | 62.68 | 57.33 | 2.20 | 1.42 |
| C. Ex. 7 | 1-MCH | 12 | 4 | 0.04 | 6.87 | 4.63 | 0.00 | 0.68 |

Rxn. Condition: (fed polymer) raw material 1, (catalyst) KOAc, (Rxn. Temp.) 153°C, (Rxn. Time) 2h Anisole: methoxy benzene, 1,2- or 1,4-DMB: 1,2- or 1,4-dimethoxybenzene, 1,3,5-TMB: 1,3,5-trimethoxybenzene, Guaiacol: 2-methoxyphenol,
Phenetole: ethoxy benezene, 1-MCH: 1-methoxycyclohexane,
BHET: bis(2-hydroxyethyl) terephthalate, Dimer: dimer of BHET,
MHET: mono(hydroxyethyl) terephthalate, KOAc : potassium acetate

In these Examples, glycolysis depolymerization was conducted in the same procedures as in Example 2, with the exception that different additives, instead of anisole, were fed in an amount of 4 moles per mole of the polymer repeating unit. The effects of the different additives on low-temperature depolymerization were analyzed and the data thus obtained are summarized in Table 6.

Comparison was made between the results from Comparative Example 5 in which PET glycolysis was performed without adding additives, Examples 2 and 16 to 20 in which compounds bearing at least one oxygen as a linking functional group between the aromatic ring moiety and the alkyl group was added. Although there were some differences in performance, it can be seen that the addition effect of the additive according to the present disclosure was remarkably observed. According to the depolymerization performance results according to Examples 16 to 18 in which modified additives were used, when m in Chemical Formula 1 increases to 2 or more, the reactivity and selectivity of the target

product were observed to slightly decrease. In Examples 19 and 20, depolymerization was carried out in the presence of modified additives in which the number of the alkyl group bonded via an oxygen atom was not one or a functional group different from the alkyl group was additionally bonded. According to the depolymerization results of Examples 18 and 19, some deviations were observed in the reactivity and the yield of BHET, but it was found that the effect according to the present disclosure was clearly observed. In Comparative Example 7, depolymerization performance was evaluated in the presence of methoxy cyclohexane (at least one alkyl group is bonded via a linker bearing one or more oxygen atoms and the central functional moiety is not an aromatic ring, but a saturated cycloalkane compound having the same number of carbons), which is structurally similar to the additive according to the present disclosure but has a different central functional group. Reactivity and BHET selectivity were observed to be lower than those of Comparative Example 5 in which no additives were added.

In exemplary embodiments of the present disclosure, evaluation was made based on the same mole number of the additives. However, if the shape of the linking functional group is different, the interaction between raw materials and the additives may appear differently. In this regard, insufficient performance may be compensated for by adjusting the form or amounts of additives introduced into the reactants.

### 1.7 Performance Evaluation of Low-Temperature Depolymerization Using Various Catalysts

The following Examples are set forth to explain the effect on the reaction when the catalyst according to the present disclosure was changed partially or totally while maintaining the composition and reaction conditions (reaction temperature: 153°C, reaction time: 2 hours) of the reactants effective for the low-temperature glycolysis. In order to determine reaction characteristics of depolymerization, depolymerization performance was observed in the presence of metallic catalysts which are metal salts containing alkaline metal or alkali earth metal cations and counter ions selected from among carbonate, bicarbonate, and alkoxide, or organic compound catalysts containing no metals.

TABLE 7

| Ex. # | Catalyst type | Mole of Cpd. per mole of polymer (PET) repeating unit | | | Conversion rate (%) | Yield (%) | | |
|---|---|---|---|---|---|---|---|---|
| | | EG | Additive | Catalyst | | BHET | Dimer | MHET |
| Ex. 21 | Na2CO3 | 10 | 3 | 0.02 | 90.58 | 74.78 | 4.27 | 2.38 |
| **Ex.** 22 | K2CO3 | 10 | 3 | 0.02 | 90.74 | 74.86 | 4.23 | 2.24 |
| Ex. 23 | NaHCO3 | 10 | 3 | 0.02 | 40.41 | 33.98 | 1.04 | 1.66 |
| Ex. 24 | KHCO3 | 10 | 3 | 0.02 | 37.21 | 30.87 | 0.78 | 1.36 |
| Ex. 25 | MgCO3 | 10 | 3 | 0.02 | 64.47 | 53.86 | 2.54 | 0.55 |
| Ex. 26 | $CH_3OK$ | 10 | 3 | 0.02 | 49.01 | 41.48 | 1.43 | 1.40 |
| Ex. 27 | TBD | 10 | 3 | 0.02 | 99.00 | 81.81 | 5.28 | 2.18 |
| Rxn. Condition: (fed polymer) raw material 1, (additive) anisole, (Rxn. Temp.) 153°C, (Rxn. Time) 2h BHET: bis(2-hydroxyethyl) terephthalate, Dimer: dimer of BHET, MHET: mono(hydroxyethyl) terephthalate, TBD: 1,5,7-triazabicyclodec-5-ene | | | | | | | | |

In Table 7, the low-temperature (153°C) depolymerization performance is evaluated and compared when various types of catalysts described above were applied. Under the condition wherein the additives according to the present disclosure were added while the amount of all compounds added relative to the polymer was limited, the evaluation was conducted in a kinetic limited regime so that the difference in performance for each catalyst was noticeable. Glycolysis depolymerization was conducted and evaluated in the same manner as in Example 1, with the exception of using the catalyst in an amount of 0.02 moles per mole of the polymer repeating unit, ethylene glycol in an amount of about 32.30 g (10 moles per mole of the polymer repeating unit), and anisole in an amount of about 16.88 g (3 moles per mole of the polymer repeating unit). Although the result was not included, the performance of the catalysts alone was first observed by performing depolymerization at a low temperature (153 ° C) under the condition that one of the various modified catalysts or organic compounds with different counter ion forms listed in Table 7 was used, without additives according to the present disclosure. As can be predicted from the results compared and described in the foregoing, when the reaction was performed without adding an additive even if the catalyst was modified, poor reactivity and very limited BHET yield were observed, as in the performance results of Comparative Examples 4 to 6.

In Examples 21 and 22 in which metal salt catalysts composed of Group 1A metal cations and a carbonate anion

was used, high reactivity and high monomer yield were observed at similar levels to that obtained when a catalyst composed of an acetate anion was applied. In Examples 23 and 24 having the same mole number of bicarbonate as an anion, relatively low reaction performance was observed. In the case of the glycolysis reaction performed in the presence of a substituted metal carbonate catalyst composed of a Group 2A metal cation under the same conditions (Example 25), higher reactivity and higher monomer yield were observed than those obtained using the aforementioned alkali metal salt catalyst having bicarbonate as an anion. When triazabicyclodecene, which is an organic compound catalyst bearing no metals, is applied in the same moles under the same conditions (Example 27), a very effective glycolysis reaction with a conversion rate of the reaction is 99.0% or more and a BHET yield of 81.8% or more was observed.

As is understood from the previous results of the depolymerization using various modified forms of transesterification catalysts, the additive (compound represented by Chemical Formula 1) according to the present disclosure can greatly improve the glycolysis reactivity to the ester functional group-bearing polymer at low temperature (153°C) and allow a high yield of BHET at less than the boiling point of ethylene glycol.

### 1.8 Purification and Analysis of Glycol-Added Monomers Produced After Depolymerization

The following Examples are set forth to determine the physical and chemical properties, including purity, of the BHET product obtained from purification processes after depolymerization of the ester functional group-bearing polymer by the method of the present disclosure. Such processes may include the recovery and separation of the additive. As described in the foregoing Examples, the addition of additives makes it possible to perform a high-performance glycolysis reaction in a low-temperature region of 153°C or lower and allows for the introduction of a catalyst system that is difficult to use in a high-temperature region of 197°C or higher, bringing about various advantages in the purification process. For example, catalysts exhibiting optimal performance in a low-temperature region can be extended to alkali metal salts, and organic compounds that can decompose at high temperatures can be applied as catalysts without loss, so that the catalysts can be easy to purify and recover. In addition, since catalysts containing harmful metals such as zinc, lead and other heavy metals, which are mainly used in existing commercial processes, are not necessarily required, the present disclosure is expected as a recycling technology for manufacturing raw materials for high-quality eco-friendly materials or for creating a perfect circular economy for waste plastics.

In the following, glycol-added monomers were obtained through purification of reaction products from respective depolymerization reactions at a high temperature (197°C) in the absence of the additive, with zinc acetate serving as a catalyst and at a low temperature (153°C) in the presence of anisole, with acetate salt of Group 1A serving as a catalyst under the condition that the compounds suggested in the present disclosure were maintained at the same mass ratios. The monomers were evaluated for yield and purity.

As seen in Table 8, depolymerization was carried out by the method of Experimental Example 1 or 2 using the polymer raw material, reaction catalyst, and ethylene glycol at a mass ratio of 1: 0.1: 4 (when added, an additive was used twice as much as the polymer raw material). Depolymerization was carried out at a high temperature (197°C) in the absence of the additive, with zinc acetate serving as a catalyst, for Comparative Example 8 and at a low temperature (153°C) in the presence of anisole, with potassium acetate and sodium acetate serving as catalysts, respectively, for Examples 28 and 29, and depolymerization products were obtained through the same purification process as in Experimental Example 3. The purified final products were characterized through ICP and $^{1}$H-NMR. For Comparative Example 8, the product was washed through the process of Experimental Example 4, and the quality of the product (Comparative Example 9) was examined.

TABLE 8

| Ex. # | Cata lyst type | Rxn. Temp . (°C) | Mole of Cpd. per mole of polymer (PET) repeating unit | | | Purificati on | Residu al metal (mg/kg ) |
|---|---|---|---|---|---|---|---|
| | | | EG | Addit ive | Cataly st | | |
| C. Ex. 8 | Zn(O Ac)$_2$ | 197 | 12.4 | 0 | 0.088 | 1 Round of filtration & Wash | 3.4 |
| C. Ex. 9 | Zn(O Ac)$_2$ | 197 | 12.4 | 0 | 0.088 | Many rounds of filtration & wash | ND |
| Ex. 28 | K(OAc) | 153 | 12.4 | 3.56 | 0.196 | 1 Round of filtration & Wash | ND |
| Ex. 29 | Na (O Ac) | 153 | 12.4 | 3.56 | 0.234 | 1 Round of filtration & Wash | ND |
| Rxn. Condition: (fed polymer) raw material 1, (additive) anisole, (Rxn. Time) 2h (weight ratio of compounds added) PET: catalyst: EG: additive = 1:0.1:4:2<br>ND: not detected | | | | | | | |

The amounts of residual metal shown in Table 8 for Comparative Example 8 and Examples 28 to 29 were contents of metals remaining in BHET obtained through primary recrystallization of the products from the glycolysis reaction pathway of PET, as analyzed by ICP-MS and ICP-AES analysis methods with a detection limit of 1 ppm or more.

The BHET obtained through a purification process after reaction using zinc acetate was detected to contain some amount of residual metal. Zinc used as a catalyst is classified as an important mineral for the human body, but can cause digestive disorders when the dose is high or continuously exposed to the human body through the use of products such as beverage containers. Various side effects may occur by an indirect route in which zinc remaining in plastic is discharged to nature as waste, accumulated in animals and plants, and then absorbed into the human body. Therefore, a low-temperature depolymerization reaction technology based on a non-hazardous metal catalyst that can replace the conventional catalysts is advantageous in terms of environmental pollution reduction as well as commerciality of the product.

Therefore, an alkali metal salt was used as a catalyst, but in the same mass as that of the zinc acetate used in Comparative Example 8 (in a larger amount in terms of moles of metal than in Comparative Example 8) to perform reaction and purification. Referring to the results of Example 28 (or Example 29), the amount of residual metal was not detected in the final product prepared using the alkali metal salt catalyst. The metal ions themselves as constituents of the catalysts are also essential electrolytes in the human body and are less harmful than zinc. The reason for the low residual amount of metal in the products manufactured according to Examples 28 and 29 is as follows: During the purification process, the components of the catalyst have a much higher ionization degree than zinc acetate in an aqueous phase and thus easily dissociate, and due to recrystallization and physical filtration processes, only a trace amount of metals remained in the BHET product recovered in the solid phase or most amounts of metals were eluted into the aqueous phase.

The qualitative characteristics of the purified glycol-added monomers were observed by $^1$H-NMR. The spectra obtained are depicted in FIGS. 1 to 3.

$^1$H-NMR of the high-temperature glycolysis-based monomer purified by the method of Comparative Example 8 is as follows:

$^1$H-NMR (500 MHz, DMSO-$d_6$) $\delta$ (ppm): 8.12 *(s,* 4H), 4.99 (br s, 2H), 4.33 (t, 4H), 3.73 (t, 4H)

Referring to a number of prior literatures on the NMR spectra of the BHET monomer obtained from the glycolysis of PET with ethylene glycol, it is reported that the characteristic peak attributable to the hydrogen of the terminal hydroxyl groups is split into a triplet at around 4.95 ppm by the hydrogen of the ethylene group adjacent thereto and a peak in a quartet form is detected at around 3.73 ppm for the hydrogen bonded to the carbon adjacent to the hydroxyl group (c in FIGS. 2 and 3). However, in the $^1$H-NMR spectrum of the BHET monomer obtained after simple purification according to the method of Comparative Example 8, a broad single peak was observed at around 4.99 ppm and a triplet was detected at around 3.27 ppm. As shown in Table 8, such peak shifts are considered to result from the interference of the zinc acetate catalyst which remained as an impurity in BHET.

In Comparative Example 9, an additional purification method was performed to reduce the residual amount of metal in the glycol-added monomer obtained according to Comparative Example 8. In order to perform additional purification according to the method of Experimental Example 4, distilled water maintained at a constant temperature was added to the white powder product finally obtained from Comparative Example 8 and stirred for about 1 hour, followed by recovering and drying the additionally washed precipitate with the same filter as that used in Comparative Example 8. While the additional purification process was repeated in the manner shown in Experimental Example 4, BHET monomers in which any metal was no longer detected within the ICP detection limit was obtained. Thereafter, the characteristic peaks of the re-measured $^1$H NMR spectrum are as follows:

$^1$H-NMR (500 MHz, DMSO-$d_6$) $\delta$ (ppm): 8.12 *(s,* 4H), 4.97 (t, 2H), 4.33 (t, 4H), 3.73 (q, 4H)

A triplet peak was detected at around 4.97 ppm, instead of the broad single peak at about 4.99 ppm for the high-temperature glycolysis-based monomer purified by the method of Comparative Example 8 while a quartet peak, but not a triplet peak, appeared at around 3.73 ppm.

Next, glycolysis was carried out as in Example 28 (using potassium acetate) and the product was purified as BHET monomers. Without further purification, the monomers were structurally analyzed by $^1$H-NMR as in Comparative Example 8, and the observed characteristic peaks are as follows:

$^1$H-NMR (500 MHz, DMSO-$d_6$) $\delta$ (ppm): 8.12 *(s,* 4H), 4.95 (t, 2H), 4.33 (t, 4H), 3.73 (q, 4H)

For the BHET reacted, purified, and collected d in the same manner as in Example 29 (using sodium acetate), almost the same $^1$H-NMR spectra were obtained (the same procedure and method as in Example 28 was applied).

As shown in the Comparative Examples and Examples, the structural characteristics of the monomers with zinc

metal remaining therein and the high-purity monomers are distinguished. Thus, when used, the catalyst system prepared according to the present disclosure enables BHET to be obtained in high purity even through a simple purification process, allows for efficient purification of BHET, and is advantageous in terms of productivity and economy.

## 2. Extractant for Removing Color-Expressing Foreign Substance from Monomer of Ester Functional Group-Bearing Polymer and Method for Purifying Monomer Using Same

### [Raw Materials 5 and 6]

Raw material 5 was prepared by collecting only colored labels composed of PET from waste plastic containers discharged after consumption, classifying same by major constituent color, and cutting into pieces with a size of 0.5 cm or less for each side. Polyester fabrics dyed with a disperse dye were cut into flakes with an area of 1 $cm^2$ or less and the fiber flakes were used as raw material 6.

### EXPERIMENTAL EXAMPLE 5: Liquid-Liquid Extraction

After completion of the reaction, about 200 g of distilled water at 95 to 100°C was added to the filtrate obtained from filtration. Then, the filtrate in water was transferred to a container maintained at 75°C and stirred for one hour. When phase separation occurred, the lower aqueous solution layer was taken and left and cooled at room temperature.

### EXPERIMENTAL EXAMPLE 6: Liquid-Liquid Back Extraction

To the organic solution layer separated in the liquid-liquid extraction of Experimental Example 5 was added about 200 g of distilled water heated to 95 to 100°C, and the mixture was transferred to a container maintained at 75°C and stirred for one hour. When phase separation occurred, the lower aqueous solution layer was taken and left and cooled at room temperature.

### EXPERIMENTAL EXAMPLE 7: Commercialization of Depolymerization Product

After a reaction mixture containing the monomer or the aqueous solution layer extracted in Experimental Example 5 or 6 was left for 12 hours in a refrigerator maintained at 4°C, the solid phase thus crystallized was obtained by removing much moisture through physical filtration. The solid was transferred to a vacuum drier maintained at 60°C and dried for 12 hours or longer in a vacuum to afford a depolymerization product.

### EXPERIMENTAL EXAMPLE 8: Recovery of Organic Solvent and Pigment

After separation of the aqueous solution layer in Experimental Example 5 or 6, anisole was separated as a gas phase from the remaining upper phase (organic layer) using pot still and then condensed into a liquid phase. The pigment-enriched residue was obtained as a viscous semi-solid product. The products thus obtained were each quantified using a high-precision balance with a precision of $\pm$0.1 mg.

### 2.1 Preparation of High-Purity Monomer from Pigment-Containing Waste PET Film

In Comparative Examples 10 and 11, colored PET films containing pigment (yellow and green raw material 5) were used as raw materials, and depolymerization was performed using the reaction conditions and compound compositions described in Table 1 for Comparative Examples 1 and 2. For each of the produced products, monomer products were obtained through the process of Experimental Example 7 after removing oligomers in the reactant mixture through the filtration method of Experimental Example 3.

In Example 30, depolymerization was performed using the same reactant composition and reaction conditions as in Example 2 using raw material 5, after which liquid-liquid extraction was carried out according to the method of Experimental Example 5 and the aqueous phase was taken and subjected to the process of Experimental Example 7 to afford a monomer product. In Example 32, the depolymerization product obtained in Comparative Example 10 was added with about 22.5 g of anisole and about 200 g of distilled water heated to 95 to 100°C, after which the liquid-liquid extraction of Experimental Example 5 was performed and the aqueous solution phase was taken and subjected to the process of Experimental Example 7 to afford a monomer product.

In Example 33, the liquid-liquid back extraction step of Experimental Example 6 was performed using the organic layer separated in the liquid-liquid extraction process of Example 31, and the aqueous phase was taken and subjected to the process of Experimental Example 7 to afford a monomer product.

The structural characteristics of the purified monomer were analyzer using nuclear magnetic resonance (model name: Bruker Avance II 500 MHz), and the residual amount of color-expressing organic foreign matter in the depolymerized product was estimated from the measured mass value of the sample and the relative area ratio of the characteristic peaks. For use as measurement samples, the 20 mg of the purified monomer and 10 mg of the organic foreign matter were each diluted in 0.7 ml of DMSO-$d_6$ solvent.

FIG. 5 shows $^1$H-NMR spectra for bis (2-hydroxyethyl) terephthalate (BHET) monomer, which can be obtained from a glycolysis reaction using an ester functional group-bearing polymer as a raw material, and a concentrated pigment recovered from a colored PET film (prepared according to Experimental Example 8). The depolymerized products prepared through the processes of Examples 31 to 33 corresponded to BHET monomers with a high purity of 99.9% or more. The characteristic peak values on the $^1$H NMR spectra measured for dilutions of the monomer were observed to be very similar to the reference values reported in many prior literatures.

$^1$H-NMR (500 MHz, DMSO-$d_6$) $\delta$ (ppm) : 8.13 *(s,* 4H), 4.96 (t, 2H), 4.32 (t, 4H), 3.72 (q, 4H)

As previously reported, the characteristic peak attributable to the hydrogen of the terminal hydroxyl groups was split into a triplet at around 4.96 ppm by the hydrogen of the ethylene group adjacent thereto and a peak in a quartet form appeared at around 3.72 ppm for the hydrogen bonded directly to the ethylene functional group adjacent to the hydroxyl group (Corresponds to 3 and 4 of the BHET chemical formula shown in FIG. 5), without distortion.

The NMR spectrum (upper spectrum in FIG. 5) of the pigment-containing organic foreign matter extracted from the colored PET film is very complex. When the polymer depolymerized product is purified, some of the organic matter can be removed by applying only a conventional physical filtration method, but it is not easy to remove the colored organic mixture. In particular, the organic compounds characterized by chemical shift values of 0.94 and 4.69 ppm had color development characteristics similar to those of the PET film used as a starting raw material colored even though only a trace amount of the pigment was contained, and the extraction method according to the present disclosure made it possible to highly purify the depolymerized monomer product (BHET).

The mass values of the samples (organic foreign substances and purified BHET) used in the NMR spectrum were compared with the integral values of the characteristic peaks to calculate the foreign substance content, and estimated contaminant contents based on respective chemical shift values are presented in Table 9.

TABLE 9

| Ex . # | Raw materia 1 | Depolymerization method | Depolymeri -zation catalyst[a] | Purification | Product color | BHET Mola r Yiel d (%)[b] | Foreign matter (wt.%)[c] | |
|---|---|---|---|---|---|---|---|---|
| | | | | | | | Peak 1 (0.94ppm ) | Peak 2 (4.69ppm ) |
| C. Ex . 10 | PET film (yellow ) | Experimenta 1 Ex. 1 | Zn $(OAc)_2$ | Filtration | Dark yellow | 80.3 | 0.7 | 1.9 |
| C. Ex . 11 | PET film (green) | Experimenta 1 Ex. 1 | K(OAc) | Filtration | Dark green | 79.2 | 2.1 | 0.4 |
| Ex . 30 | PET film (green) | Experimenta 1 Ex. 2 | K(OAc) | Filtration , extraction | Greenis h white | 76.8 | 0.3 | 0.2 |
| Ex . 31 | PET film (green) | Experimenta 1 Ex. 2 | K(OAc) | Filtration , extraction twice | White | 74.3 | - | - |
| Ex . 32 | Low-density BHET (yellow ) | - | - | Filtration , extraction | White | 75.4 | - | - |
| Ex . 33 | Organic -phase reactio n mixture (green) | Experimenta 1 Ex. 2 | K(OAc) | Filtration , extraction , and back extraction | White | 1.7 | - | - |

a. $Zn(OAc)_2$: zince acetate, KOAc: potassium acetate
b. yield in terms of number of moles of BHET product relative to number of moles of starting PET repeating unit (%)
c. concentration of foreign matter, calculated from integral value of $^1$H-NMR peak ($\delta$ = 0.94, 4.69 ppm)

The yield of the depolymerized product was calculated as a ratio (%) of the number of moles of the depolymerized product obtained from each step to the amount of product quantified by HPLC, based on the total number of moles of the repeating units in the polymer introduced as the initial raw material. The amounts of depolymerized monomer products recovered according to Comparative Examples 10 and 11 and Examples 30 to 33 using pigment-containing waste PET film as a raw material are presented as BHET molar yield (%) in Table 9. As the extraction process was repeated two or more times, the content of foreign substances was very low, but the yield of the final product decreased with the increase of the mass of monomers lost during the product purification process.

### 2.2 Preparation of High-Purity Monomers from Dye-Containing Polyester Fibers

Unlike pigments that are fixed in a particle or powder state after being dispersed in a solvent, dyes are used in a state dissolved in a solvent and mainly aim to dye textile materials or leather. In particular, a dye in the form of an organic compound containing at least one aromatic ring is generally used for coloring a fiber material such as ester functional group-bearing polymer fiber, for example, polyester.

As the dye, a material having a very high affinity for the terephthalate functional group as a constituent of the fiber is used as a colorant to prevent discoloration from the polymer fiber, and should be physically and chemically dispersed uniformly in the fiber. As a result, even if depolymerization proceeds, it may not be easy to separate and purify high-purity products due to high interaction with monomers or low molecular weight compounds produced by depolymerization. Therefore, there is some miscibility with the product monomer or low-molecular compound. Nonetheless, supply of a large amount of solvents having strong affinity for organic dyes and addition of excessive hydrophilic solvents lead to asymmetric solute (dye) distribution, whereby high-purity products can be recovered.

In Comparative Example 12, a monomer product was obtained in the same manner as in Comparative Example 11, with the exception of using raw material 6 (dark blue waste polyester fibers). In Example 34, a monomer product was obtained in the same manner as in Example 31, with the exception of using raw material 6.

In Example 35, the liquid-liquid back extraction of Experimental Example 6 was performed using the organic layer separated in the repeated liquid-liquid extraction process of Example 34 as a raw material, and then the aqueous phase was taken and subjected to the process of Experimental Example 7 to afford a monomer product.

In Example 36, the liquid-liquid extraction process of Example 34 was repeated four times, and then the aqueous phase was taken and subjected to the process of Experimental Example 7 to afford a monomer product.

FIG. 6 shows NMR spectra of BHET monomers obtained by depolymerizing colored polyester fibers as raw materials and for the dye concentrated and recovered according to Experimental Example 8.

In the $^{1}$H-NMR spectra obtained for the products manufactured according to the methods of Examples 34 to 36, main peaks were clearly detected at the intrinsic chemical shift values responsible for BHET monomer structural characteristics, as in the spectra obtained from Examples 30 to 33 in which raw materials including pigments were used. However, delimitation of the number of extractions or additional recovery of the product through back extraction could increase the overall yield of the depolymerized product, but the purity of the BHET monomer was limited to less than 99%, and the removal of foreign substances did not proceed effectively due to the dye of intrinsic color included therein. As a result of observing the monomer products manufactured according to the Experimental Examples of the present disclosure, even if the organic dye remained in a trace amount of 1% by mass or less, it could be easily determined with the naked eye that the product contained foreign substance.

In order to recover BHET of the same quality as the high-purity monomer product (Examples 31 to 33) by purifying the reaction mixture obtained from the depolymerization of the waste PET raw materials containing the pigment stated in the foregoing, a number of iterative extraction processes as in the purification process of Example 36 is required.

Table 10 shows specific examples of processes for obtaining high-purity monomers from depolymerization reaction mixtures obtained using colored polyester raw materials. It was found that the organic disperse dye responsible for the color can be efficiently removed by adding the compound represented by Chemical Formula 1 and applying the extraction and purification process (Experimental Example 5). When two rounds of the extraction and purification process (Example 34) and two rounds of back extraction using the organic phases generated in the two rounds of the extraction process (Example 35) were performed, products with a purity of 99.5% or more were obtained as powders which were pale reddish due to the trace amount of residual dye. After four or more rounds of the extraction and purification process, the final product was produced at a yield of as low as about 51%, but the residual dye was detected at a level of 0.1% or less, so that white BHET products in high purity could be obtained.

TABLE 10

| Ex. # | Raw material | Depolymerization method | Depolymerization catalyst[a] | Purification | Product color | BHET Molar Yield %)[b] | Foreign matter (wt.%)c | | |
|---|---|---|---|---|---|---|---|---|---|
| | | | | | | | Peak 1 (3.56ppm) | Peak 2 (4.52ppm) | Peak 3 (8.06ppm) |
| C. Ex. 12 | Polyester fiber (dark blue) | Experimental Ex. 2 | K(OAc) | Filtration | Dark red | 75.2 | > 5.0 | > 6.2 | > 4.7 |
| Ex. 34 | Polyester fiber (dark blue) | Experimental Ex. 2 | K(OAc) | Filtration, extraction twice | Pale red | 68.2 | 0.37 | 0.24 | 0.16 |
| Ex. 35 | Organic phase rxn. mixture (black) | Experimental Ex. 2 | K(OAc) | Filtration, extraction twice and back extraction | Pale red | 4.3 | 0.39 | 0.26 | 0.17 |
| Ex. 36 | Polyester fiber (dark blue) | Experimental Ex. 2 | K(OAc) | Filtration, extraction 4 times | White | 51.3 | - | - | - |

a. $Zn(OAc)_2$: zince acetate, KOAc: potassium acetate

b. yield in terms of number of moles of BHET product relative to number of moles of starting PET repeating unit (%)

c. concentration of foreign matter, calculated from integral value of $^1H$-NMR peak ($\delta$ = 3.56, 4.52, 8.06 ppm)

Although the specific part of the present disclosure has been described in detail, it is obvious to those skilled in the art that such a specific description is just a preferred embodiment and the scope of the present disclosure is not limited. Thus, the substantial scope of the present disclosure will be defined by the appended claims and equivalents thereof.

**Industrial Applicability**

The present disclosure relates to a method for depolymerizing an ester functional group-bearing polymer with or without a pigment or dye included therein, a composition for the depolymerization, and a purification method for selectively separating foreign substances from monomers of the polymer. The present disclosure can find advantageous applications in the technical fields of preparing, recovering, and reusing ester functional group-bearing polymers and thus is industrially applicable.

Further, the following embodiments are disclosed:

1. A method for depolymerization of an ester functional group-bearing polymer, the method comprising contacting with the ester functional group-bearing polymer a mixture comprising:

(A) at least one polyhydric alcohol;
(B) a transesterification catalyst; and
(C) a compound represented by the following Chemical Formula 1:

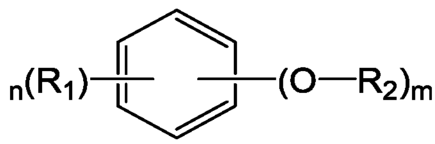

[Chemical Formula 1]

wherein,

$R_1$ is any one selected from the group consisting of a hydrogen atom, a hydroxy, an aldehyde, a carboxyl, an alkyl of $C_1$-$C_6$, a cycloalkyl of $C_4$-$C_6$, and an aryl of $C_6$-$C_{12}$;
n is an integer of 0 to 5 wherein when n is 2 or more, the corresponding $R_1$'s are same or different;
$R_2$ is an alkyl of $C_1$-$C_{10}$; and
m is an integer of 1 to 6 wherein when m is 2 or more, the corresponding -O-$R_2$'s are same or different.

2. The method of 1, wherein the transesterification catalyst is an organic compound catalyst including no metals, or at least one metal salt selected from among salts of metals in groups 1A, 2A, and 2B.

3. The method of 2, wherein the metal salt comprises a counter ion composed of an organic anion selected from among carbonate, bicarbonate, alkoxide, and acetate.

4. The method of 1, wherein the polyhydric alcohol is at least one selected from among ethylene glycol, diethylene glycol, triethylene glycol, propylene glycol, dipropylene glycol, 1,3-butanediol, 1,4-butanediol, 3-methyl-1,5-pentanediol, 1,6-hexanediol, 1,7-octanediol, 1,9-nonanediol, neopentyl glycol, 1,4-cyclohexanediol, isosorbide, and 1,4-cyclohexane dimethanol.

5. The method of 1, wherein the ester functional group-bearing polymer is at least one selected from the group consisting of polyethylene terephthalate (PET), polypropyleneterephthalate(PPT), polyglycolide 또는 polyglycolic acid (PGA), polylactic acid (PLA), polycaprolactone (PCL), polyhydroxyalkanoate (PHA), polyhydroxybutyrate (PHB), polyethylene adipate (PEA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), and Vectran.

6. The method of 1, wherein the compound represented by Chemical Formula 1 is at least one selected from the group consisting of methoxybenzene, 1,2-dimethoxybenzene, 1,3-dimethoxybenzene, 1,4-dimethoxybenzene, 1,2,3-trimethoxybenzene, 1,2,4-trimethoxybenzene, 1,3,5-trimethoxybenzene, 1,2,3,4-tetramethoxybenzene, 1,2,3,5-tetramethoxybenzene, 1,2,4,5-tetramethoxybenzene, 2-methoxyphenol, 1,3-dimethoxy-2-hydroxybenzene, 4-hydroxy-3,5-dimethoxybenzoic acid, 3-(4-hydroxy-3,5-dimethoxyphenyl)prop-2-enal, 4-hydroxy-3,5-dimethoxybenzaldehyde, 4-hydroxy-3,5-dimethoxyacetophenone, 3-(4-hydroxy-3,5-dimethoxyphenylprop-2-enoic acid, 4-enyl-2,6-dimethoxyphenol, 4-hydroxy-3-methoxybenzaldehyde, 3-hydroxy-4-methoxybenzaldehyde, 2-hydroxy-3-methoxybenzaldehyde, 2-hydroxy-5-methoxybenzaldehyde, 2-hydroxy-4-methoxybenzaldehyde, 3,4-dihydroxybenzaldehyde, 1-methoxy-4-[(E)-prop-1-en-yl]benzene, 1-bromo-4-methoxybenzene, 2-tert-butyl-4-methoxyphenol, ethoxybenzene, 1,3,5-trichloro-2-methoxybenzene, 1,3,5-tribromo-2-methoxybenzene, 4-(prop-2-en-1-yl)phenol, 1-methoxy-4(prop-2-en-1-yl)benzene, 5-(prop-2-en-1-yl)-2H-1,3-benzodioxole, 4-methoxy-2-[(E)-prop-1-yl]phenol, and 2-methoxy-4-(prop-1-en-yl)phenol.

7. A composition for depolymerizing an ester functional group-bearing polymer, the composition comprising:

(A) at least one polyhydric alcohol;
(B) a transesterification catalyst; and
(C) a compound represented by Chemical Formula 1,

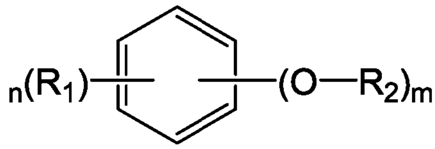

[Chemical Formula 1]

wherein,

$R_1$ is any one selected from the group consisting of a hydrogen atom, a hydroxy, an aldehyde, a carboxyl, an alkyl of $C_1$-$C_6$, a cycloalkyl of $C_4$-$C_6$, and an aryl of $C_6$-$C_{12}$;
n is an integer of 0 to 5 wherein when n is 2 or more, the corresponding $R_1$'s are same or different;
$R_2$ is an alkyl of $C_1$-$C_{10}$; and
m is an integer of 1 to 6 wherein when m is 2 or more, the corresponding -O-$R_2$'s are same or different.

8. The composition of 7, wherein the transesterification catalyst is an organic compound catalyst including no metals, or at least one metal salt selected from among salts of metals in groups 1A, 2A, and 2B.

9. The composition of 7, wherein the composition contains the polyhydric alcohol in an amount of 1 to 50 moles per mole of the repeating unit of the polymer to be depolymerized, the transesterification catalyst in an amount of 0.001 to 1 mole per mole of the repeating unit of the polymer to be depolymerized, and the compound represented by Chemical Formula 1 in an amount of 0.001 to 50 moles per mole of the repeating unit of the polymer to be depolymerized.

10. A method for purifying a glycol-added monomer obtained by the depolymerization method of any one of 1 to 6, the method comprising the steps of:

(1) separating the compound represented by Chemical Formula 1 from the reaction mixture of depolymerization through gas-liquid separation or liquid-liquid extraction,

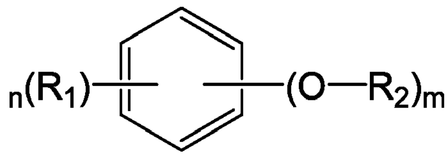

[Chemical Formula 1]

wherein,

$R_1$ is any one selected from the group consisting of a hydrogen atom, a hydroxy, an aldehyde, a carboxyl, an alkyl of $C_1$-$C_6$, a cycloalkyl of $C_4$-$C_6$, and an aryl of $C_6$-$C_{12}$;
n is an integer of 0 to 5 wherein when n is 2 or more, the corresponding $R_1$'s are same or different;
$R_2$ is an alkyl of $C_1$-$C_{10}$; and
m is an integer of 1 to 6 wherein when m is 2 or more, the corresponding -O-$R_2$'s are same or different;

(2) separating a solid containing the ester functional group-bearing polymer remaining reacted after step (1);

(3) recovering the glycol-added monomer through recrystallization after the solid separating step (2).

11. The method of 10, wherein the solid including an unreacted ester functional group-bearing polymer is separated by physical filtration.

12. The method of 10, wherein a step of separating an oligomer may be carried out prior to the recrystallization of the glycol-added monomer in step (3).

## Claims

1. An extractant for removing a color-expressing foreign matter from a monomer of an ester functional group-bearing polymer resin, the extractant including a compound represented by Chemical Formula 1:

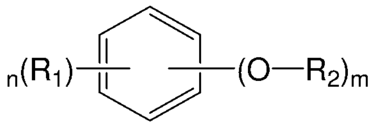
[Chemical Formula 1]

(wherein,

$R_1$ is any one selected from the group consisting of a hydrogen atom, a hydroxy, an aldehyde, a carboxyl, an alkyl of $C_1$-$C_6$, a cycloalkyl of $C_4$-$C_6$, and an aryl of $C_6$-$C_{12}$;
n is an integer of 0 to 5 wherein when n is 2 or more, the corresponding $R_1$'s are same or different;
$R_2$ is an alkyl of $C_1$-$C_{10}$; and
m is an integer of 1 to 6 wherein when m is 2 or more, the corresponding -O-$R_2$'s are same or different.)

2. The extractant of claim 1, wherein the compound represented by Chemical Formula 1 is at least one selected from the group consisting of methoxybenzene, 1,2-dimethoxybenzene, 1,3-dimethoxybenzene, 1,4-dimethoxybenzene, 1,2,3-trimethoxybenzene, 1,2,4-trimethoxybenzene, 1,3,5-trimethoxybenzene, 1,2,3,4-tetramethoxybenzene, 1,2,3,5-tetramethoxybenzene, 1,2,4,5-tetramethoxybenzene, 2-methoxyphenol, 1,3-dimethoxy-2-hydroxybenzene, 4-hydroxy-3,5-dimethoxybenzoic acid, 3-(4-hydroxy-3,5-dimethoxyphenyl)prop-2-enal, 4-hydroxy-3,5-dimethoxybenzaldehyde, 4-hydroxy-3,5-dimethoxyacetophenone, 3-(4-hydroxy-3,5-dimethoxyphenylprop-2-enoic acid, 4-enyl-2,6-dimethoxyphenol, 4-hydroxy-3-methoxybenzaldehyde, 3-hydroxy-4-methoxybenzaldehyde, 2-hydroxy-3-methoxybenzaldehyde, 2-hydroxy-5-methoxybenzaldehyde, 2-hydroxy-4-methoxybenzaldehyde, 3,4-dihydroxybenzaldehyde, 1-methoxy-4-[(E)-prop-1-en-yl]benzene, 1-bromo-4-methoxybenzene, 2-tert-butyl-4-methoxyphenol, ethoxybenzene, 1,3,5-trichloro-2-methoxybenzene, 1,3,5-tribromo-2-methoxybenzene, 4-(prop-2-en-1-yl)phenol, 1-methoxy-4(prop-2-en-1-yl)benzene, 5-(prop-2-en-1-yl)-2H-1,3-benzodioxole, 4-methoxy-2-[(E)-prop-1-yl]phenol, and 2-methoxy-4-(prop-1-en-yl)phenol.

3. The extractant of claim 1, wherein the monomer of the ester functional group-bearing polymer is obtained by depolymerizing the ester functional group-bearing polymer.

4. A purification method for producing a monomer in high purity by removing foreign matter from a monomer of an ester functional group-bearing polymer resin, the method comprising the steps of:

1) adding a compound represented by the following Chemical Formula 1 to a monomer of an ester functional group-bearing polymer with a color-expressing foreign matter incorporated thereto and mixing same;
2) leaving the mixture of step 1) to induce liquid-liquid phase separation; and
3) separately discharging the upper organic solution phase and the lower aqueous solution phase in the phase-separated liquid-liquid layers,

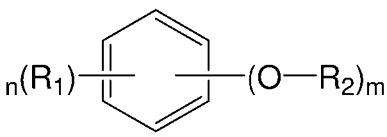
[Chemical Formula 1]

(wherein,

$R_1$ is any one selected from the group consisting of a hydrogen atom, a hydroxy, an aldehyde, a carboxyl, an alkyl of $C_1$-$C_6$, a cycloalkyl of $C_4$-$C_6$, and an aryl of $C_6$-$C_{12}$;
n is an integer of 0 to 5 wherein when n is 2 or more, the corresponding $R_1$'s are same or different;
$R_2$ is an alkyl of $C_1$-$C_{10}$; and
m is an integer of 1 to 6 wherein when m is 2 or more, the corresponding -O-$R_2$'s are same or different.)

**5.** The purification method of claim 4, wherein the step 1) comprises adding water or a hydrophilic liquid immiscible with the compound represented by Chemical Formula 1 to the monomer having a color-expressing foreign matter incorporated thereinto.

**6.** The purification method of claim 4, wherein the monomer is a reaction product from depolymerization of the ester functional group-bearing polymer or is obtained by partially purifying a mixture from resulting the depolymerization.

**7.** The purification method of claim 4, wherein the method further comprises a step of carrying one or more rounds of further adding the compound represented by Chemical Formula 1 to the separated aqueous solution layer to extract the color-expressing foreign matter.

**8.** The purification method of claim 4, wherein the method further comprises a step of adding water or a hydrophilic liquid immiscible with the compound represented by Chemical Formula 1 to the separated organic solution layer and mixing same to perform phase separation again.

**9.** The purification method of claim 4, wherein the method further comprises a step of recovering the monomer of the ester functional group-bearing polymer resin from the separated aqueous solution phase through recrystallization.

**10.** A purification method for preparing a monomer in high purity, the method comprising the steps of:

(a) adding a compound of Chemical Formula 1 to an ester functional group-bearing colored polymer;
(b) depolymerizing the ester functional group-bearing polymer;
(c) adding and mixing the reaction mixture obtained by the depolymerization with water or a hydrophilic solution immiscible with the compound represented by Chemical Formula 1;
(d) leaving the mixture of step (c) to induce liquid-liquid phase separation; and
(e) separately discharging the upper organic solution layer and the lower aqueous solution layer in the phase-separated liquid-liquid layers, whereby color-expressing foreign matter is removed from the monomer obtained by depolymerization of the ester functional group-bearing colored polymer,

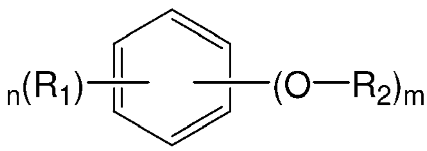

[Chemical Formula 1]

(wherein,

$R_1$ is any one selected from the group consisting of a hydrogen atom, a hydroxy, an aldehyde, a carboxyl, an alkyl of $C_1$-$C_6$, a cycloalkyl of $C_4$-$C_6$, and an aryl of $C_6$-$C_{12}$;
n is an integer of 0 to 5 wherein when n is 2 or more, the corresponding $R_1$'s are same or different;
$R_2$ is an alkyl of $C_1$-$C_{10}$; and
m is an integer of 1 to 6 wherein when m is 2 or more, the corresponding -O-$R_2$'s are same or different.)

**11.** The purification method of claim 10, wherein the method further comprises a step of recovering and recycling the organic solvent from the separately discharged organic solution layer in step (e) through distillation.

FIG. 1

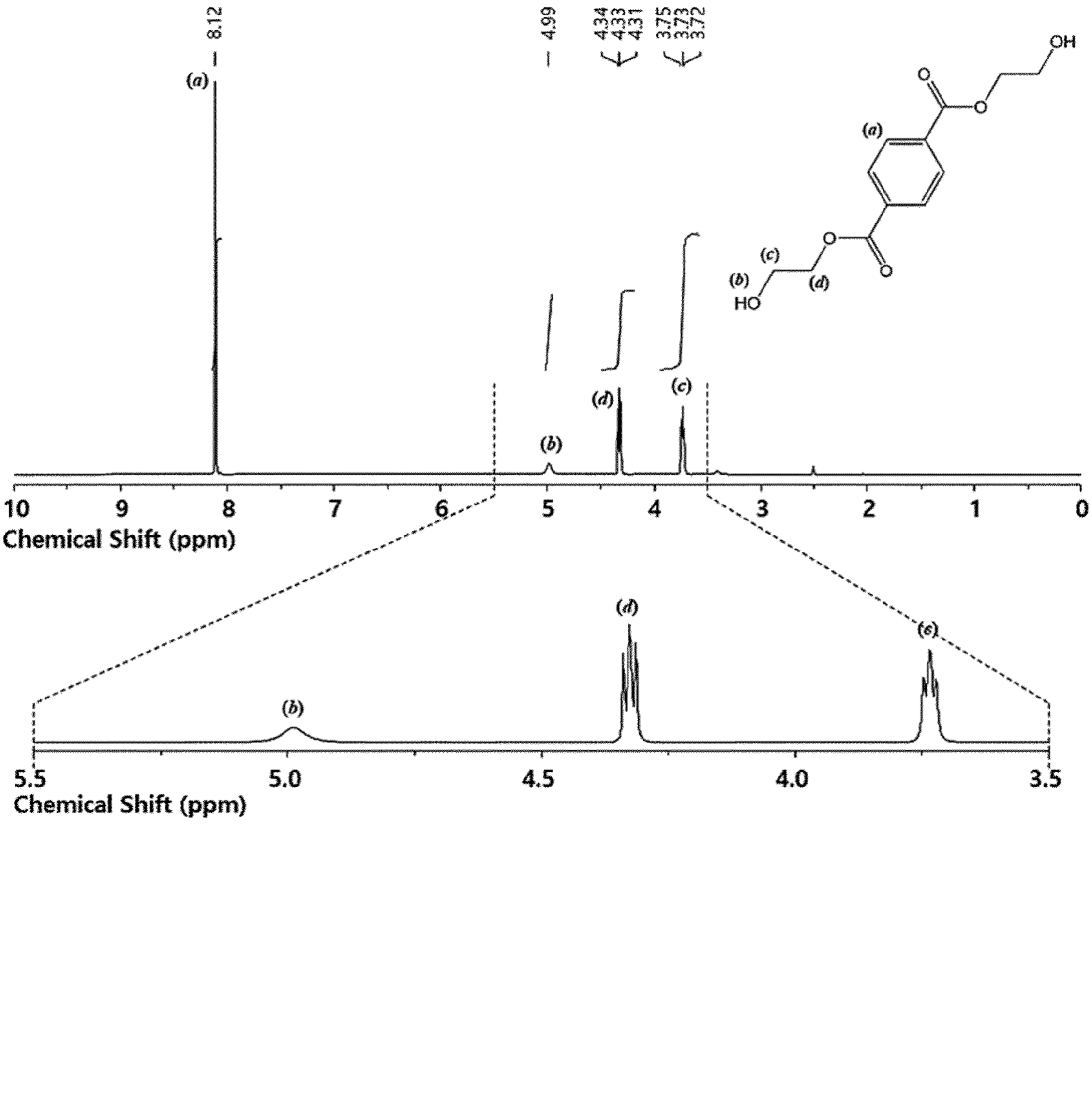
8.12
4.99
4.34
4.33
4.31
3.75
3.73
3.72
(a)
(b)
(c)
(d)
10 9 8 7 6 5 4 3 2 1 0
Chemical Shift (ppm)
(b)
(d)
(c)
5.5 5.0 4.5 4.0 3.5
Chemical Shift (ppm)

FIG. 2

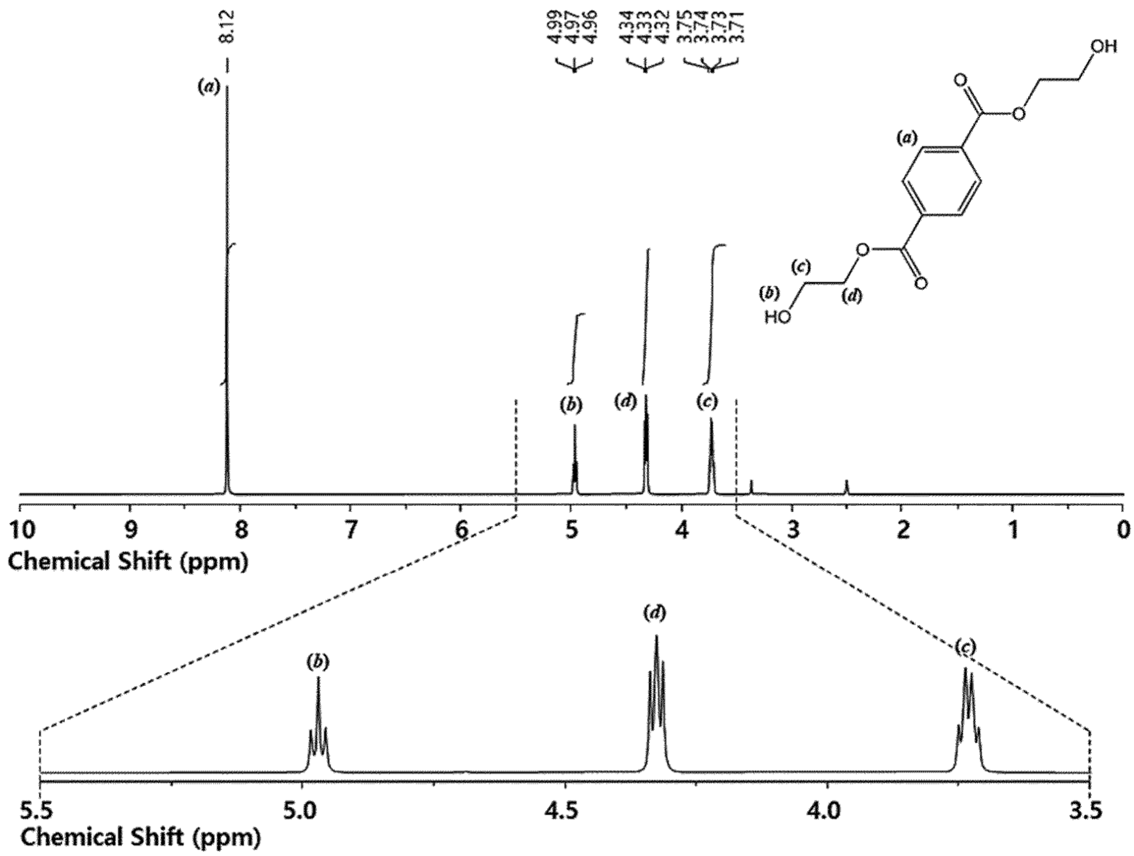
8.12
4.99
4.97
4.96
4.34
4.33
4.32
3.75
3.74
3.73
3.71
(a)
(b)
(d)
(c)
OH
HO
10
9
8
7
6
5
4
3
2
1
0
Chemical Shift (ppm)
5.5
5.0
4.5
4.0
3.5
Chemical Shift (ppm)

FIG. 3

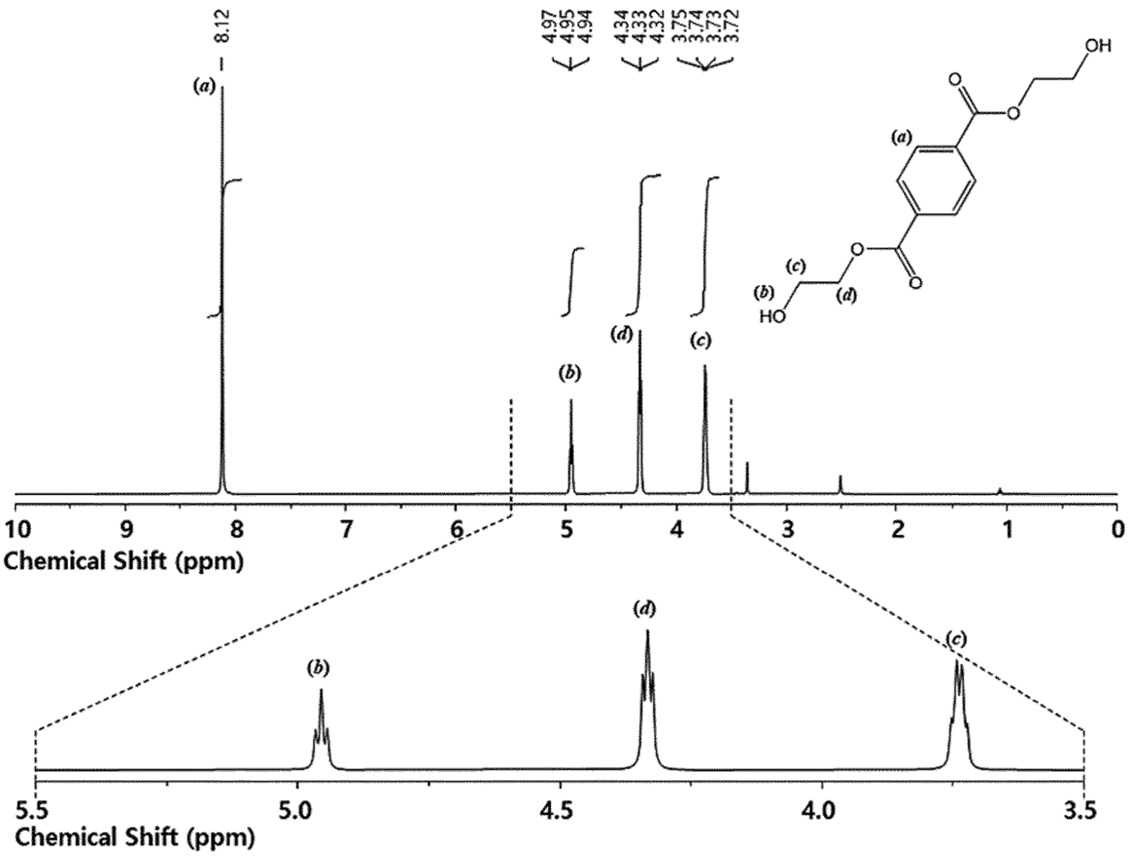
8.12
4.97
4.95
4.94
4.34
4.33
4.32
3.75
3.74
3.73
3.72
(a)
(b)
(c)
(d)
10
9
8
7
6
5
4
3
2
1
0
Chemical Shift (ppm)
5.5
5.0
4.5
4.0
3.5
Chemical Shift (ppm)

FIG. 4

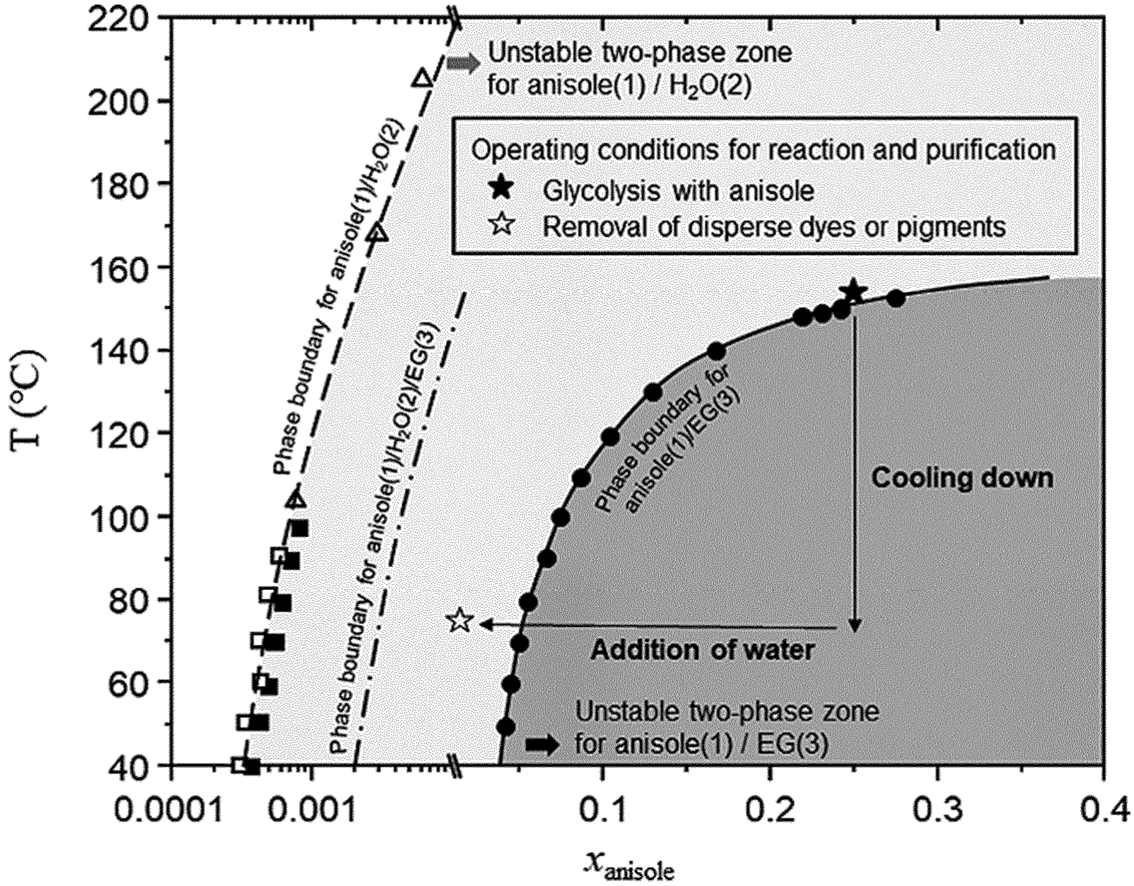

220
200
180
160
140
120
100
80
60
40
T (°C)
0.0001
0.001
0.1
0.2
0.3
0.4
$x_{anisole}$
Unstable two-phase zone for anisole(1) / $H_2O$(2)
Operating conditions for reaction and purification
Glycolysis with anisole
Removal of disperse dyes or pigments
Phase boundary for anisole(1)/$H_2O$(2)
Phase boundary for anisole(1)/$H_2O$(2)/EG(3)
Phase boundary for anisole(1)/EG(3)
Cooling down
Addition of water
Unstable two-phase zone for anisole(1) / EG(3)

FIG. 5

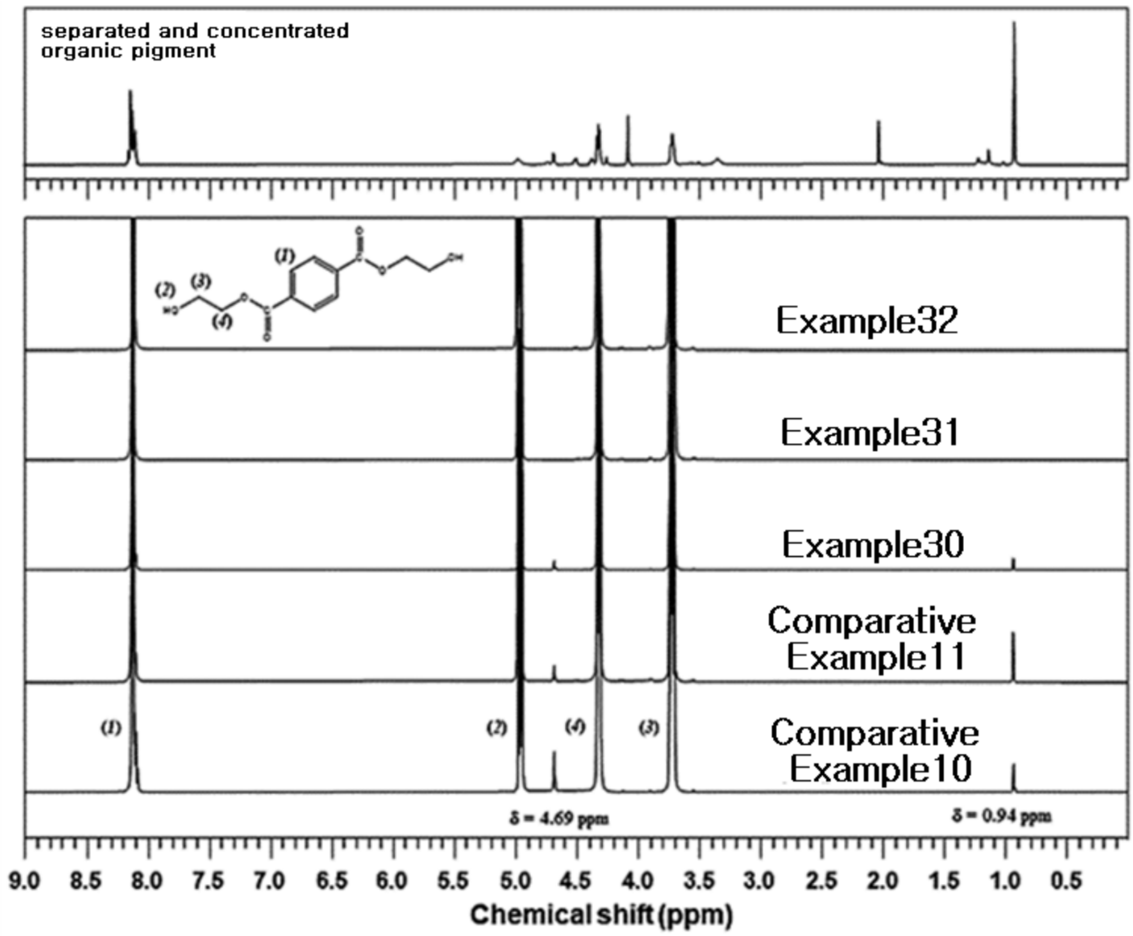
separated and concentrated
organic pigment
(1)
(2) (3)
(4)
Example32
Example31
Example30
Comparative
Example11
(1)
(2)
(4)
(3)
Comparative
Example10
δ = 4.69 ppm
δ = 0.94 ppm
9.0 8.5 8.0 7.5 7.0 6.5 6.0 5.5 5.0 4.5 4.0 3.5 3.0 2.5 2.0 1.5 1.0 0.5
Chemical shift (ppm)

FIG. 6

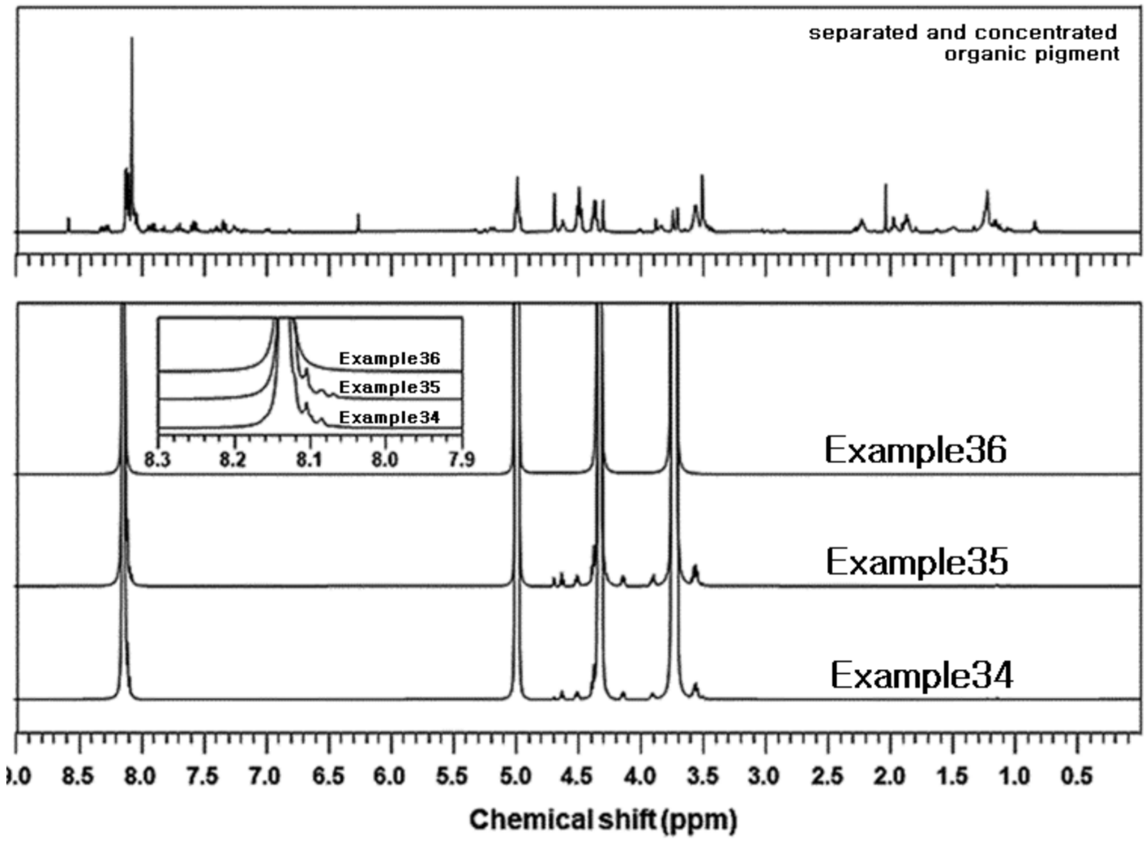
separated and concentrated organic pigment
Example36
Example35
Example34
8.3
8.2
8.1
8.0
7.9
Example36
Example35
Example34
9.0
8.5
8.0
7.5
7.0
6.5
6.0
5.5
5.0
4.5
4.0
3.5
3.0
2.5
2.0
1.5
1.0
0.5
Chemical shift (ppm)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 201201228480 A
- KR 1142328
- JP 4537288 B
- KR 101142329